# FASCICULE DE BREVET EUROPEEN

(11) **EP 2 785 682 B1**
(45) Date de publication et mention de la délivrance du brevet: **03.04.2019**
(21) Numéro de dépôt: 12810327.2
(22) Date de dépôt: 03.12.2012
(51) Int. Cl.: C07C 253/22, C07C 227/04, C07C 229/08, C07C 253/30, C07C 255/19, C07C 255/17, C07C 255/07

(54) **PROCEDE DE PREPARATION D'AMINOACIDE COMPRENANT UNE ETAPE D'HYDROFORMYLATION D'UN NITRILE GRAS INSATURE**
AMINOSÄURENHERSTELLUNGSVERFAHREN MIT EINEM SCHRITT ZUR HYDROFORMYLIERUNG EINES UNGESÄTTIGTEN FETTSÄURENITRILS
AMINO ACID PREPARATION METHOD COMPRISING A STEP OF HYDROFORMYLATION OF AN UNSATURATED FATTY NITRILE

(30) Priorité: 01.12.2011 FR 1161036
(43) Date de publication de la demande: 08.10.2014
(73) Titulaire: Arkema France, 92700 Colombes (FR); Centre National de la Recherche Scientifique, 75794 Paris Cedex 14 (FR); Université de Rennes 1, 35065 Rennes Cedex (FR)
(72) Inventeur: DUBOIS, Jean-Luc, F-69390 Millery (FR); COUTURIER, Jean-Luc, F-69006 Lyon (FR); CARPENTIER, Jean-François, F-35690 Acigne (FR); TERNEL, Jérémy, F-32710 Courrieres (FR)
(74) Mandataire: Schaefer, Anne-Sophie
(86) Numéro de dépôt international: PCT/FR2012/052778
(87) Numéro de publication internationale: WO 2013/079888

(56) Documents cités:
- WO-A1-97/33854
- WO-A1-2010/055273

## Description

### Domaine de l'invention :

La présente invention porte sur un nouveau procédé de synthèse d'acides ω-aminoalcanoïques susceptibles d'être utilisés dans l'industrie des polymères, notamment des polyamides, ledit procédé comprenant une étape d'hydroformylation d'un nitrile gras insaturé.
Par « nitrile gras insaturé », on entend tout composé de formule *(1)*: CH₂=CH-(CH₂)ᵣ-CN dans laquelle 4 ≤ r≤ 13.
On sait aujourd'hui fabriquer ces composés nitriles gras insaturés à partir de composés acides ou esters gras insaturés, ou à partir de composés saturés comportant une fonction hydroxyle, pouvant être aussi bien d'origine fossile que d'origine renouvelable. Par acides ω-aminoalcanoïques, ci-après « ω-aminoacides » ou simplement « aminoacides », au sens de l'invention, on entend en fait tout ω-aminoacide à chaîne longue, c'est-à-dire dont la chaîne comporte au moins 8 atomes de carbones.
En effet, les polyamides visés par la présente invention sont des polyamides techniques, c'est-à-dire des polyamides de performance, de haute performance, voire de très haute performance, fabriqués à partir de monomères comportant au moins 8 atomes de carbone, de préférence au moins 10 atomes de carbones; par opposition aux polyamides dits de « commodité », tels que le « nylon 6 », dont les quantités (volumes) commercialisées sont très supérieures et les coûts très inférieurs à ceux des polyamides techniques.

### Technique antérieure :

L'industrie des polyamides utilise toute une gamme de monomères formés à partir de diamines et de diacides, de lactames, et surtout à partir d' ω-aminoacides. Ces derniers sont définis par la longueur de chaîne méthylène (-CH₂)ₙ séparant deux fonctions amide -CO-NH-. Ces monomères sont généralement fabriqués par voie de synthèse chimique en utilisant comme matières premières des oléfines en C2 à C4, des cycloalcanes ou du benzène, hydrocarbures issus de sources fossiles. Par exemple, les oléfines en C2 servent à fabriquer l'aminoacide en C9 utilisé dans le Pelargon russe ; les oléfines en C4 servent à fabriquer l'héxaméthylène diamine ; le laurolactame et le caprolactame sont fabriqués à partir de cycloalcanes ; l'acide adipique, le Nylon 6 et le Nylon 6,6 sont fabriqués à partir de benzène.

Concernant la préparation de polyamides à partir de composés nitriles insaturés, le brevet US7026473 décrit l'hydroxycarbonylation ou la méthoxycarbonylation, d'un pentène-nitrile en acide ou ester 5-cyanovalérique (6 atomes de C), en présence de CO (monoxyde de carbone) et respectivement d'eau ou d'alcool. Seule la méthoxycarbonylation avec le méthanol est en fait exemplifiée. Par réduction, l'acide (ester) 5-cyanovalérique forme l'acide (ester) 6-aminocaproïque, qui à son tour donne l'ε-caprolactame par cyclisation (c'est le monomère du Nylon-6). Le procédé décrit dans ce document présente plusieurs inconvénients. L'étape de méthoxycarbonylation est lente et coûteuse en catalyseur. La conversion n'est pas totale et nécessite des temps de réaction élevés. De plus, de nombreux co-produits sont formés, en particulier les produits branchés, qui doivent être séparés du produit linéaire que l'on cherche à produire. Ce document ne concerne pas un procédé comprenant une étape d'hydroformylation, ni la fabrication d'aminoacides de nombre de carbone au moins égal à 8. Le procédé décrit utilise des composés nitriles à chaine courte, 5 carbones, pour fabriquer des produits à 6 carbones aux propriétés chimiques très différentes de celles recherchées par la présente invention. Par ailleurs, ce document ne concerne pas la fabrication d'aminoacides bio-ressourcés.

Le document de brevet WO97/33854 décrit un procédé de fabrication d'aldéhyde terminal par hydroformylation d'un alcène, tel que l'hexène, le butadiène, le méthyl-3-penténoate, le 3-pentènenitrile. Ce document montre qu'il est beaucoup plus difficile d'obtenir un aldéhyde linéaire (faible proportion de linéaires obtenus) à partir d'un nitrile (3-pentènenitrile) qu'à partir d'un ester. De plus, dans le cas d'hydroformylation à partir de nitrile de l'art antérieur, on obtient une importante proportion (21%, 16,3%) de produit réduit (valéronitrile) c'est-à-dire ne contenant plus d'aldéhyde, à cause de l'hydrogénation de la double liaison par le catalyseur. En outre, l'obtention de produits linéaires dans l'art antérieur se fait au détriment de la conversion. De nouveau, ce document ne concerne pas la fabrication d'aminoacides de nombre de carbone au moins égal à 8. Le procédé décrit utilise des composés nitriles à chaine courte, 5 carbones, pour fabriquer des produits à 6 carbones aux propriétés chimiques très différentes de celles recherchées par la présente invention. Par ailleurs, ce document ne concerne pas la fabrication d'aminoacides bio-ressourcés.

L'évolution actuelle en matière d'environnement conduit dans les domaines de l'énergie et de la chimie à privilégier l'exploitation de matières premières naturelles, provenant de source renouvelable. Seuls quelques monomères sont fabriqués à ce jour à partir de matières premières bio-ressourcées, comme l'huile de ricin, qui permet de fabriquer le polyamide 11 commercialisé sous la marque Rilsan®; l'huile érucique qui permet de fabriquer le polyamide 13/13, ou encore l'huile lesquérolique qui permet de fabriquer le polyamide 13.
Typiquement, le procédé de fabrication de l'acide 11-aminoundécanoïque comprend les étapes suivantes :
1) Alcoolyse (méthanolyse) de l'huile de ricin en ricinoléate de méthyle (RM),
2) Craquage (pyrolyse) du RM,
3) Distillation pour récupérer l'undécylénate de méthyle,
4) Hydrolyse de l'undécylénate de méthyle en acide undécylénique
5) Hydrobromuration pour obtenir l'acide 11-bromo-undécanoïque,
6) ammonolyse par une solution aqueuse ammoniacale pour conduire à l'acide 11-aminoundécanoïque.
La polycondensation de l'acide 11-aminoundécanoïque, par polymérisation hydrolytique en présence d'acide phosphorique comme catalyseur, permet ensuite d'obtenir le polyamide 11.
Le procédé de synthèse de l'acide 11-aminoundécanoïque, opéré de manière industrielle depuis plusieurs décennies donne globalement satisfaction. Cependant, il présente un certain nombre d'inconvénients. Le premier inconvénient est que sa mise en oeuvre est en pratique asservie à l'accès à une seule matière première spécifique, l'huile de ricin. De plus, la graine de ricin contient une toxine : la ricine, extrêmement toxique. Le deuxième inconvénient est lié à certains réactifs utilisés, l'ammoniac et le brome en particulier, qui exigent des précautions de stockage et d'utilisation coûteuses, et nécessitent d'investir dans des unités spécifiques de séparation et de recyclage du bromure d'ammonium formé. Le troisième inconvénient est lié aux co-produits obtenus par le procédé : du glycérol mais aussi de nombreux sous-produits qu'il faut valoriser séparément, tels que l'heptanaldéhyde, l'estérol : mélange d'esters d'acides gras non craqués.

Le document US6307108 (voir en particulier colonne 9 lignes 25-58) décrit la fabrication du 12-aminododécanoate de méthyle à partir de l'undécylénate de méthyle (UM), issu de l'huile de ricin. Le procédé comprend une étape d'hydroformylation de l'UM pour former l'aldéhyde-ester en C12, puis une étape d'amination réductrice pour fabriquer l'amino-ester en C12. Pour pouvoir fabriquer l'aminoacide correspondant, ce procédé impose une étape supplémentaire d'hydrolyse de l'aminoester dans des conditions qui ont comme inconvénient d'entraîner sa polycondensation directe en « polyamide » dont la croissance de chaine est limitée par la présence des groupements esters.

La présente invention a donc pour but de trouver un nouveau procédé de synthèse directe d' ω-aminoacides, faisant intervenir d'autres matières premières et réactifs, qui évite la formation de sous-produits, qui minimise le nombre d'étapes, et qui ne présente pas les inconvénients précités.

La présente invention a encore pour but de trouver un nouveau procédé de synthèse de l'ensemble de la gamme des ω-aminoacides à chaîne longue, simple à mettre en oeuvre, et utilisant autant que possible des matières premières renouvelables, de préférence accessibles largement.

Par matières premières renouvelables accessibles « largement », on entend celles qui sont aisément disponibles, par exemple issues de plantes déjà cultivées et/ou faciles à cultiver, en quantité compatible avec une production industrielle, et peu onéreuses.

Dans cette voie « bio », la demanderesse a déjà décrit plusieurs procédés de synthèse d'ω-aminoacides à partir de nitriles gras insaturés d'origine renouvelable. Les documents de brevet WO2010055273, FR11.55174, FR11.56526, et FR11.57542 décrivent notamment les étapes de synthèse d'un ω-aminoacide à partir de nitrile gras insaturé par coupure oxydante ou par métathèse croisée avec un acrylate conduisant à un nitrile-acide, puis son hydrogénation en aminoacide. Ces procédés comprenant une métathèse utilisant des catalyseurs et des co-matières premières relativement coûteux, telles que l'acrylonitrile ou l'acrylate de méthyle. En particulier, les ligands du ruthénium utilisés pour catalyser la métathèse sont très spécifiques et constituent la part essentielle du prix du catalyseur. D'autre part, sur tous les carbones de l'aminoacide formé par ces procédés, au moins 2 carbones ne sont pas bio-ressourcés si l'acrylate de méthyle ou l'acrylonitrile ne sont pas eux-mêmes bio-ressourcés, ce que l'on cherche encore à améliorer, le but étant de fabriquer des aminoacides qui tendent vers le 100 % renouvelable. Par ailleurs, dans le document WO2010055273 notamment, le procédé comprend une ozonolyse contrôlée, ce qui revient à enlever un carbone à la chaîne carbonée alors qu'on cherche au contraire, dans le cas de la synthèse de polyamides de spécialités, à augmenter la longueur de la chaîne carbonée des monomères fabriqués. De plus, le procédé de métathèse dans les documents précités conduit non pas à un aminoacide mais à un aminoester dans les cas où les matières premières sont par exemple un ester gras et de l'acrylonitrile, ou bien un nitrile gras et de l'acrylate de méthyle. Pour produire un polyamide conventionnel, l'aminoester doit préalablement être reconverti en aminoacide, ce qui nécessite une étape supplémentaire très complexe pour hydrolyser à chaud une fonction ester sans démarrer la polymérisation de l'aminoester.

La présente invention a donc également pour but de trouver un nouveau procédé utilisant des catalyseurs, en particulier des ligands, et des co-matières plus simples et moins coûteux, et permettent d'augmenter la teneur en matière d'origine renouvelable des aminoacides.

La Demanderesse a maintenant trouvé un nouveau procédé de synthèse faisant intervenir une étape d'hydroformylation d'un nitrile gras insaturé, avec de l'hydrogène (H₂) et du monoxyde de carbone (CO), et ne comportant pas les inconvénients précités.

### Description détaillée de l'invention :

Dans la présente description, on précise que lorsqu'il est fait référence à des intervalles, les expressions du type « allant de...à » ou « comportant/comprenant de... à » incluent les bornes de l'intervalle. A l'inverse, les expressions du type « compris entre...et... » excluent les bornes de l'intervalle.

Sauf mention contraire, les pourcentages exprimés sont des pourcentages molaires. Sauf mention contraire, les paramètres auxquels il est fait référence sont mesurés à pression atmosphérique.

La présente invention a donc pour objet un procédé de synthèse d'un composé ω-aminoacide de formule HOOC-(CH₂)ᵣ₊₂-CH₂NH₂ comprenant :
1) une étape d'hydroformylation d'un nitrile gras insaturé choisi parmi les composés de formule: CH₂=CH-(CH₂)ᵣ-CN où 4 ≤ r ≤ 13,
   et leurs mélanges,
   en faisant réagir ledit nitrile avec du monoxyde de carbone et du dihydrogène de façon à obtenir au moins un aldéhyde nitrile gras de formule :

   HOC-(CH₂)ᵣ₊₂-CN
2) une étape d'oxydation en présence de dioxygène (oxygène moléculaire), au cours de laquelle l'aldéhyde nitrile obtenu à l'étape 1 est converti en acide nitrile gras de formule :

   HOOC-(CH₂)ᵣ₊₂-CN
3) une étape de réduction au cours de laquelle l'acide nitrile obtenu à l'étape 2 est converti en ω-aminoacide de formule :

   HOOC-(CH₂)ᵣ₊₂-CH₂NH₂.

Le nouveau procédé de l'invention dans lequel on effectue une étape d'hydroformylation d'un nitrile gras insaturé, suivie d'une étape d'oxydation de l'aldéhyde ainsi formé en acide, puis d'une étape de réduction de la fonction nitrile en fonction amine, aboutit à la synthèse directe d'aminoacides.

Avantageusement, le procédé selon l'invention comprend en outre une étape de métathèse catalytique croisée avec un alcène choisi parmi l'éthylène réalisée sur le nitrile gras avant l'étape 1) de façon à fabriquer un nitrile gras oméga-insaturé répondant à la formule : R₂-CH=CH-(CH₂)ᵣ-CN, où R₂ est H.

Cette étape de métathèse catalytique croisée avec un alcène est réalisée classiquement dans les mêmes conditions que celles décrites par exemple dans la demande de brevet WO2010055273 page 9 lignes 12 à 18.

Le nitrile gras insaturé de départ utilisé dans le procédé selon l'invention est généralement obtenu à partir de composés acides ou esters gras insaturés (ou hydroxylés) par nitrilation (ammoniation) d'au moins une fonction acide ou ester de ces composés qui peuvent être issus de matières premières d'origine fossile ou d'origine renouvelable.

Les composés acides ou esters gras insaturés peuvent être obtenus par exemple selon le procédé décrit par le document de brevet US4510331. Ce dernier décrit en particulier la fabrication de l'acide 7-octénoïque, par isomérisation du 2,7-octadièn-1-ol en 7-octén-1-al, puis oxydation de ce dernier en acide. Le 2,7-octadiène-1-ol est produit industriellement par réaction (« télomérisation ») du butadiène avec de l'eau en présence de catalyseur au palladium selon le procédé décrit dans les documents de brevet GB2074156A et DE3112213. Ce type de procédé utilise des matières premières d'origine fossile.

De manière alternative, les nitriles gras insaturés sont fabriqués à partir d'acides ou d'esters gras insaturés d'origine renouvelable, issus des huiles naturelles. Ces procédés développés récemment par Arkema sont décrits notamment dans les documents de brevet : WO2010055273, FR11.55174, FR11.56526, et FR11.57542.

Par nitrile gras insaturé au sens de l'invention, on entend de préférence ceux obtenus au moins partiellement à partir d'acides gras naturels insaturés. Avantageusement, le procédé selon l'invention comprend donc une étape de fabrication dudit nitrile gras à partir d'un acide ou ester gras insaturé d'origine naturelle de formule :

(CH₂₌CH-(CH₂)ᵣ-COO)ₚ-G

dans laquelle p est un indice entier tel que 1≤ p≤ 3, G est H (un hydrogène), un radical alkyle de 1 à 11 atomes de carbone ou un radical comportant 2 ou 3 atomes de carbone porteur de 1 ou 2 fonctions(s) hydroxyle(s), la(les) double(s) liaison(s) C=C pouvant être en conformation cis ou trans, ladite fabrication comprenant :
- l'ammoniation (action qui consiste à introduire de l'ammoniac dans un produit) de la fonction carbonyle de l'acide (ou ester) gras insaturé d'origine naturelle en fonction nitrile.

Le schéma réactionnel de la synthèse des nitriles à partir des acides, par ammoniation (ou nitrilation, les deux termes étant utilisés indifféremment), bien connue de l'homme de l'art, peut se résumer de la façon suivante.

R-COOH + NH₃ → [R-COO-NH₄+] → [R-CONH₂] + H₂O → RCN + H₂O

Ce schéma s'applique tout aussi bien aux acides (esters) gras naturels qu'aux acides gras ω-insaturés. Le procédé peut être conduit en batch en phase liquide ou gazeuse ou en continu en phase gazeuse. La réaction est conduite à température élevée supérieure à 250 °C et en présence d'un catalyseur qui est généralement un oxyde métallique et le plus fréquemment l'oxyde de zinc. L'élimination en continu de l'eau formée en entraînant de surcroît l'ammoniac qui n'a pas réagi permet un achèvement rapide de la réaction.

L'ammoniation en phase liquide est bien adaptée pour les chaînes grasses longues (comportant au moins 10 atomes de carbone). Cependant, en opérant avec des longueurs de chaîne plus courtes, l'ammoniation en phase gaz peut devenir plus appropriée. Il est également connu de GB 641,955 de réaliser l'ammoniation en utilisant comme agent l'urée ou l'acide cyanurique. On peut aussi utiliser toute autre source d'ammoniac.

Selon un mode de réalisation particulier, le nitrile gras insaturé utilisé selon l'invention est fabriqué à partir d'acides gras naturels à longue chaîne insaturés. On entend par acide gras naturel à longue chaîne un acide issu du milieu végétal ou du milieu animal, y compris les algues ou d'autres micro-organismes, et donc renouvelable, comportant de 6 à 24 atomes de carbone, avec de préférence au moins 7 (si l'aminoacide final a au moins 8 C) atomes de carbone, de préférence au moins 8 atomes de carbone, de préférence au moins 10 atomes de carbone, et de préférence au moins 14 atomes de carbone par molécule. Ces divers acides sont issus des huiles végétales extraites de diverses plantes telles que le tournesol, le colza, la cameline, le ricin, le lesquerella, l'olive, le soja, le palmier, la coriandre, le céleri, l'aneth, la carotte, le fenouil, le Limnanthes Alba (meadowfoam). Ils sont issus également du monde animal terrestre ou marin, et dans ce dernier cas, aussi bien sous forme de poissons, de mammifères que d'algues. Il s'agit en général de graisses provenant de ruminants, de poissons comme la morue, ou de mammifères marins comme les baleines ou les dauphins.

Comme acide gras insaturé convenant plus particulièrement à la mise en oeuvre de l'invention, on peut citer : l'acide pétrosélénique (acide cis-6-octadécénoïque), son dérivé l'acide 6-hepténoïque obtenu par éthénolyse (métathèse croisée avec l'éthylène), l'acide α-linolénique (6-9-12-octadécatriénoïque), ces acides pouvant être obtenus à partir de la coriandre par exemple ; l'acide cis-8-eicosénoïque, l'acide cis-5,8,11,14- eicosatriénoïque (acide arachidonique), l'acide ricinoléique qui donne après déshydratation l'acide 8,10-octadécadiènoïque conjugué ; l'acide caproléique (cis-9-décénoïque), l'acide palmitoléique (cis-9- hexadécénoïque), l'acide myristoléique (cis-9-tétradécénoïque), l'acide oléique (cis-9-octadécénoïque), l'acide 9-décénoique obtenu par éthénolyse d'un acide oléique par exemple, l'acide élaidique (trans-9-octadécénoïque), l'acide ricinoléique (12-hydroxy-cis-9-octadécénoïque), l'acide gadoléique (cis-9-eicosénoïque), l'acide linoléique (9-12-octadécadiénoïque), l'acide ruménique (9-11-octadécadiénoïque), l'acide linoléique conjugué (9-11-octadécadiénoïque), ces acides pouvant être obtenus à partir de tournesol, colza, ricin, olive, soja, palmier, lin, avocat, argousier, coriandre, céleri, aneth, carotte, fenouil, Limnanthes (meadowfoam) ; l'acide linoléique conjugué 10-12 (10-12-octadécadiénoïque), l'acide 10 undécylénique obtenu par craquage thermique de l'ester méthylique de l'acide ricinoléique par exemple; l'acide vaccénique (cis-11-octadécénoïque), l'acide gondoïque (cis-11-eicosénoïque), l'acide lesquérolique (14-hydroxy-cis-11-eicosénoïque), l'acide cétoléique (cis-11-docosénoïque), pouvant être obtenus à partir de l'huile de Lesquerella (lesquérolique), de l'huile de Camelina sativa (gondoïque), de l'huile d'une plante de la famille des sapindaceae, de la graisse de poisson, des huiles de microalgues (cétoléique), par déshydratation de l'acide 12-hydroxystéarique lui-même obtenu par hydrogénation d'acide ricinoléique (acide vaccénique et son équivalent trans), de l'acide linoléique conjugué (9-11-octadécadiénoïque), obtenu par exemple par déshydratation de l'acide ricinoléique ; l'acide 12-octadécénoïque (cis ou trans) obtenu par exemple par déshydratation de l'acide 12-hydroxystéarique (abrégé 12HSA) lui-même obtenu par hydrogénation de l'acide ricinoléique, l'acide linoléique conjugué 10-12 (10-12-octadécadiénoïque), l'acide 12-tridécénoïque obtenu par craquage thermique de l'ester (méthylique notamment) de l'acide lesquérolique ; l'acide érucique (cis-13-docosénoïque) et brassidique (trans-13-docosénoïque) qui peuvent par exemple être obtenus à partir du colza érucique, de la lunaire ou du crambe maritime (choux marin) ; l'acide 13-eicosenoique (cis ou trans) obtenu par déshydratation de l'acide 14-hydroxyeicosanoique lui-même obtenu par hydrogénation de l'acide lesquerolique, L'acide 14-eicosenoique (cis ou trans) obtenu par déshydratation de l'acide 14-hydroxyeicosanoique (abrégé 14HEA) lui-même obtenu par hydrogénation de l'acide lesquerolique (la déshydratation peut se faire des deux côtés du OH), l'acide nervonique (cis-15-tétracosoïque) qui peut être obtenu à partir de Malania oleifera et de lunaire (lunaria annua aussi connue sous le nom de monnaie du pape, honesty ou money plant en anglais) ; ou leurs mélanges. Il est aussi possible de s'affranchir de l'étape de déshydratation des acides 12HSA et 14HEA en conduisant la conversion en nitrile directement sur ces acides gras saturés et hydroxylés, comme décrit dans le document de brevet ayant pour numéro de dépôt FR11.56526. Un avantage de cette solution est que l'hydrogénation de l'acide ricinoléique en mélange avec les autres acides gras de l'huile de ricin conduit à un mélange ne contenant plus comme espèces majoritaires que l'acide 12HSA, l'acide stéarique et l'acide palmitique. La déshydratation suivant (ou simultanée à) la conversion en nitrile conduit à un nitrile très propre contenant plus de 85 % de nitrile monoinsaturé. Il en est de même avec le 14HEA, comme décrit dans le document de brevet FR11.56526.

Parmi les acides gras insaturés précités, on préfère ceux qui sont les plus abondamment disponibles, et en particulier les acides gras insaturés en position δ-9 ou δ-10 en numérotant à partir du groupement acide. On privilégie en effet l'utilisation de nitriles et d'acides gras comportant de 10 à 24 atomes de carbone, et de préférence ceux comportant 10 carbones ou 11 carbones avec une insaturation en position omega ou ω, c'est-à-dire en fin de chaîne par rapport au groupement acide. On privilégie par exemple les acides gras à 18 carbones comportant une insaturation en position δ-9 ou 10 par rapport au groupement nitrile ou acide, c'est-à-dire en position ω-9 ou 8 respectivement qui par éthénolyse conduiront à des acide ω-insaturés, ainsi que l'acide ricinoléique qui par craquage thermique de son ester méthylique donne l'ester méthylique de l'acide undécylénique.

Les acides gras cités ci-dessus peuvent être isolés par toutes les techniques bien connues de l'homme du métier : distillation moléculaire, y compris à court temps de trajet (« short path distillation » en anglais), cristallisation, extraction liquide-liquide, complexation à l'urée, y compris extraction au CO₂ supercritique, et/ou toute combinaison de ces techniques.

Selon un mode de réalisation particulier du procédé de l'invention, le nitrile gras insaturé est obtenu à partir d'un ester d'acide gras, ce dernier pouvant avantageusement être choisi parmi les esters des acides gras précités, notamment leurs esters méthyliques. Les voies d'obtention d'un nitrile gras à partir d'un ester d'acide gras sont par exemple décrites dans le document WO2010089512.

Selon un autre mode de réalisation, le nitrile gras insaturé est obtenu à partir d'un hydroxyacide gras, tel que 12HSA et 14HEA. Plus généralement, l'hydroxyacide gras peut avantageusement être choisi parmi ceux décrits dans la demande de brevet ayant pour numéro de dépôt FR11.56526.

De manière alternative, le nitrile gras insaturé est obtenu à partir d'un triglycéride, ce dernier pouvant avantageusement être choisi parmi : une huile végétale comprenant un mélange de triglycérides d'acides gras insaturés, telle que l'huile de tournesol, colza, ricin, lesquerella, cameline, olive, soja, palmier, sapindaceae (sapindacées) en particulier d'avocat, argousier, coriandre, céleri, aneth, carotte, fenouil, mangue, Limnanthes Alba (meadowfoam) et leurs mélanges ; des micro-algues ; des graisses animales.

Selon un autre mode de réalisation, le nitrile gras insaturé est obtenu à partir d'une cire végétale, par exemple de jojoba.

Avantageusement, le procédé selon l'invention comprend en outre, avant l'étape d'ammoniation décrite ci-dessus :
- soit une métathèse catalytique croisée avec de l'éthylène (ou autre alpha oléfine légère en C2 à C4) réalisée sur l'acide (ou l'ester ou le triglycéride) gras insaturé d'origine naturelle
- soit une pyrolyse de l'acide ou ester gras insaturé d'origine naturelle suivie de distillation (puis éventuellement hydrolyse en acide dans le cas de l'ester).
de façon à fabriquer un acide (ou ester) gras oméga-insaturé de formule : CH₂=CH-(CH₂)ᵣ-COOR₂, R₂ est H ou un radical alkyle en C1-4,
et de sorte qu'à l'issue de l'étape d'ammoniation réalisée sur cet acide (ou ester) gras oméga-insaturé, on obtient un nitrile gras oméga-insaturé de formule : CH₂=CH-(CH₂)ᵣ-CN.

L'obtention d'un tel nitrile gras insaturé à partir d'un acide/ester gras insaturé est notamment décrite dans la demande de brevet WO201005527, en particulier aux paragraphes décrivant le « premier stade » du procédé objet de ce document : c'est-à-dire à la page 5 lignes 12 à 32, à la page 7 lignes 17 à 26, page 8 lignes 1 à 9, page 10 ligne 29 à page 11 ligne 19.

Selon un mode de réalisation particulier du procédé de l'invention, on utilise un nitrile ω-insaturé de formule CH₂=CH-(CH₂)ᵣ-CN obtenu par transformation d'un acide/ester gras insaturé en en deux étapes successives (d'ordre indifférent) : éthénolyse (métathèse croisée avec l'éthylène) et ammoniation, telles que décrites dans le document WO2010055273. Selon une autre variante du procédé, des acides gras hydroxylés sont utilisés comme matière première, tels que l'acide ricinoléique et l'acide lesquérolique qui répondent à la formule générale R₁-CH=CH-(CH₂)ᵣ-COOH avec R₁ égal à CH₃-(CH₂)₅CHOH-CH₂- et r égal respectivement 7 et 9. On soumet l'acide sous sa forme ester méthylique à une pyrolyse conduisant à un ester ω-insaturé de formule CH₂=CH-(CH₂)ᵣ₊₁-COOCH₃ qui est transformé par ammoniation, directement ou en passant par l'acide, en un nitrile ω-insaturé. Selon encore un autre mode de réalisation, le nitrile gras insaturé est fabriqué comme décrit dans le document FR11.55174, par ammoniation d'un composé de type acide, ester ou glycéride gras conduisant au nitrile insaturé correspondant. Selon un mode de réalisation particulier du procédé de l'invention, on effectue comme dans le procédé du document FR11.56526, l'hydrogénation d'acides gras hydroxylés insaturés comportant au moins 18 atomes de carbone par molécule, conduisant à des acides gras hydroxylés saturés, suivie de leur déshydratation conduisant à des acides gras mono-insaturés, avec en outre soit une étape intermédiaire de nitrilation de la fonction acide de l'acide gras mono-insaturé conduisant à un nitrile insaturé, soit une étape intermédiaire de nitrilation de la fonction acide de l'acide gras hydroxylé saturé issu de l'étape d'hydrogénation avec déshydratation concomitante conduisant à un nitrile gras insaturé. Des conditions particulières d'obtention de nitriles gras insaturés sont décrites dans le document FR11.57542, comprenant la nitrilation d'un acide/ester ω-insaturé de formule CH₂=CH-(CH₂)ᵣ-COOR dans laquelle r est 7 ou 8 et R soit H, soit un radical alkyle comportant 1 à 4 atomes de carbone, par action de l'ammoniac dans un réacteur fonctionnant en continu en phase gaz ou en phase mixte gaz-liquide en présence d'un catalyseur solide.

Qu'il s'agisse de la métathèse sur le nitrile gras ou bien sur l'acide (ou l'ester gras) insaturé, la réaction de métathèse croisée avec un alcène, tel que l'éthylène, mise en oeuvre dans certaines variantes du procédé de l'invention, est conduite à une température comprise entre 20 et 100 °C à une pression de 1 à 30 bars en présence d'un catalyseur de métathèse classique par exemple de type Ruthénium. Le temps de réaction est choisi en fonction des réactifs mis en oeuvre et pour atteindre au plus près l'équilibre de la réaction. La réaction est effectuée sous une pression d'alcène.

La réaction de pyrolyse mise en oeuvre dans une variante du procédé de l'invention est réalisée de préférence sur la forme ester de l'acide gras concerné, en général l'ester méthylique. La réaction est conduite à haute température, comprise entre 400 et 750 °C et de préférence entre 500 et 600 °C en présence de vapeur d'eau surchauffée.

De préférence, l'acide de départ est un acide hydroxylé, et il s'agit de préférence de l'acide ricinoléique ou de l'acide lesquerollique, l'acide ricinoléique étant préféré car il conduit à l'acide 12 aminododécanoïque selon le procédé de l'invention.

Le procédé selon l'invention consiste donc en un procédé de synthèse d'un composé ω-aminoacide de formule

HOOC-(CH₂)ᵣ₊₂-CH₂NH₂,

à partir d'un composé nitrile gras mono-insaturé de formule

CH₂=CH-(CH₂)ᵣ-CN

comportant les étapes suivantes :
- l'hydroformylation du composé nitrile insaturé pour obtenir un composé aldéhyde-nitrile de formule HOC-(CH₂)ᵣ₊₂-CN, puis
- l'oxydation du composé aldéhyde-nitrile pour obtenir le composé acide-nitrile correspondant de formule

   HOOC-(CH₂)ᵣ₊₂-CN,

   et
- la réduction du composé acide-nitrile en ω-aminoacide de formule

   HOOC-(CH₂)ᵣ₊₂-CH₂NH₂.

### 1) HYDROFORMYLATION

L'hydroformylation, appelé aussi procédé oxo, est une voie de synthèse pour produire des aldéhydes à partir d'alcènes découverte en 1938 par Otto Roelen de chez Ruhrchemie. La réaction de base est la suivante:

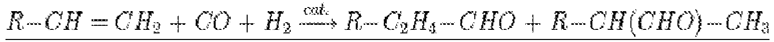

Ce procédé est largement utilisé industriellement pour produire des aldéhydes dans un intervalle de C3-C19. Le butanal est d'ailleurs le principal produit synthétisé par cette réaction avec environ 75% de la production totale utilisant l'hydroformylation comme voie de synthèse. L'étape d'hydroformylation selon le procédé de l'invention utilise les méthodes et dispositifs bien connus et déjà employés par les procédés conventionnels d'hydroformylation. Tous les modes usuels d'ajout et de mélange des réactifs et des composants de catalyseur(s), de même que les techniques de séparation usuelles pour la réaction d'hydroformylation classique peuvent donc être utilisés pour cette étape du procédé de l'invention. L'étape d'hydroformylation selon le procédé de l'invention a l'avantage de pouvoir être utilisée directement dans les nombreux dispositifs existants. Ce ne serait pas le cas de la méthoxycarbonylation ni de l'hydroxycarbonylation par exemple.

L'étape d'hydroformylation est catalysée en présence d'un système catalyseur comprenant :
- au moins un métal choisi parmi le rhodium, le palladium, le cobalt, le ruthénium et leurs mélanges; et
- au moins un ligand bidentate sélectionné pour la sélectivité de la réaction d'hydroformylation en faveur de l'aldéhyde linéaire, choisi parmi une diphosphine chélatante, ou un ligand monodentate de type monophosphine ou monophosphite.

Le ratio molaire [ligand]/[métal] est avantageusement compris dans la gamme de 60:1 à 1:1, de préférence de 40:1 à 5:1, de préférence de 30:1 à 10:1, de préférence de 20:1 à 10:1.

De préférence, le système comprend au moins une diphosphine chélatante ou une monophosphine ou monophosphite choisie parmi : Dppm, Dppe, Dppb, Xantphos, PPh₃, P(OPh)₃, de préférence Xantphos.

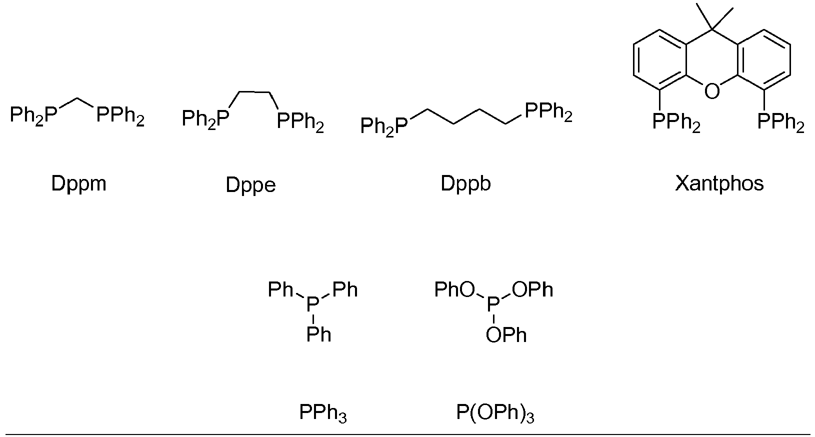

Avantageusement, le métal du système est apporté sous la forme d'un précurseur comprenant ledit métal et au moins un composé choisi parmi des acétylacétonates, des halogénures, des composés carbonyles, et leurs mélanges.

Les catalyseurs à base de rhodium sont préférés, ils améliorent sensiblement la conversion. Les catalyseurs en rhodium ont une meilleure sélectivité pour les aldéhydes, entraînent moins d'hydrogénation comme réaction parallèle et offrent des rapports produits linéaires/produits ramifiés nettement en faveur des produits linéaires. De manière avantageuse, le système catalyseur d'hydroformylation comprend du rhodium, apporté de préférence par un précurseur à base de Rh(l) tel que Rh(acac)(CO)₂, où acac est un ligand acétylacétonate et CO est un ligand carbonyle, et éventuellement un autre précurseur métallique tel qu'un précurseur à base de ruthénium, apporté par exemple sous la forme Ru₃(CO)₁₂. L'hydroformylation est catalysée de préférence par un système choisi parmi : Rh-PPh₃, Rh-P(OPh)₃, Rh-Xantphos, et leurs mélanges.

Plusieurs stratégies permettent de faire déplacer une double liaison du nitrile gras insaturé en position terminale où se fait la carbonlylation. On peut utiliser un double système catalyseur, dans lequel un premier catalyseur au cobalt ou ruthénium, qui isomérise l'alcène interne en alcène terminal, intervient parallèlement au second catalyseur au rhodium utilisé pour l'hydroformylation. Selon un autre mode de réalisation, la présence d'acide méthane sulfonique (AMS) en plus du système catalyseur utilisé selon l'invention permet également d'orienter la double liaison en position terminale. Selon un autre mode de réalisation, un second ligand, en plus de ceux précités, par exemple la biphephos, est ajouté au précurseur de rhodium pour générer une espèce catalytique qui assure en parallèle l'isomérisation dynamique de la double liaison. Ainsi, le procédé de l'invention permet d'utiliser des nitriles à double liaison interne, et d'améliorer les rendements en produits linéaires.

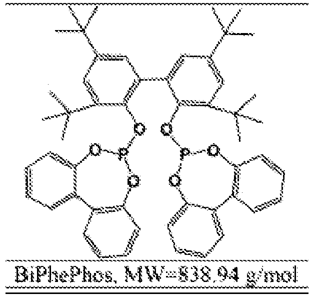

L'hydroformylation est réalisée de préférence en milieu organique, de préférence en solution dans le toluène, mais peut aussi être réalisée sans solvant. Le milieu peut également être aqueux, par exemple dans le cas où le procédé utilise du propylène au cours d'une étape de métathèse, mais il y a un risque de problème de solubilité des produits.

Selon le procédé de la présente invention, l'hydroformylation est réalisée à une température comprise dans la gamme de 70 à 150°C, de préférence de 100 à 140°C, de préférence de 120 à 140°C, de préférence à une température sensiblement égale à 140 °C.

De préférence, l'étape d'hydroformylation est réalisée pendant une durée allant de 2 à 24 heures, de préférence de 2 à 6 heures.

L'hydroformylation est réalisée de préférence sous pression partielle de CO/H₂ comprise dans la gamme de 5 à 50 bar, de préférence de 10 à 40 bar, de préférence de 10 à 30 bar, de préférence sous 20 bar de CO/H₂ et selon un ratio CO:H₂ compris dans la gamme de 1:3 à 3:1, de préférence sensiblement égal à 1:1. On préfère éviter d'utiliser trop d'H₂. Un gaz de synthèse type a un ratio CO:H₂ de 1:2 à 1:3 lorsqu'il vient de gaz naturel, et plutôt de 2:1 à 1:2 lorsqu'il vient de biomasse. Il est particulièrement intéressant de valoriser des gaz de synthèse pauvres en hydrogène, issus de la biomasse.

Selon le procédé selon l'invention, le ratio [Substrat]/[Métal] est avantageusement compris dans la gamme de 5 000 à 500 000, de préférence de 5 000 à 400 000, de préférence de 5 000 à 300 000, de préférence de 5 000 à 200 000, de préférence de 5000 à 150 000.

Selon un mode de réalisation particulier du procédé de l'invention, l'étape d'hydroformylation comprend le recyclage du système catalyseur d'hydroformylation, éventuellement complété par un apport de catalyseur neuf (ou « frais ») et/ou de ligand neuf (ou « frais ») lors d'un cycle ultérieur d'hydroformylation.

De préférence, le système catalyseur recyclé est obtenu par évaporation au moins partielle de solvant et/ou d'aldéhyde-nitrile et ou de réactif n'ayant pas réagi contenu par exemple dans le résidu de la distillation. Le catalyseur est alors recyclé au réacteur d'hydroformylation, avec ajout éventuel de Phosphine et/ou de Rhodium et/ou purge du résidu de la distillation.

### 2) OXYDATION (OU AUTO-OXYDATION, OU « AUTOXYDATION »)

A l'issue de l'étape d'hydroformylation, l'aldéhyde-nitrile obtenu présente l'avantage de s'oxyder très facilement au contact de dioxygène.

Selon un mode de réalisation préféré, l'étape d'oxydation du procédé selon l'invention est mise en oeuvre en faisant barboter du dioxygène ou un mélange gazeux contenant du dioxygène dans le produit résultant de l'hydroformylation, éventuellement en présence du catalyseur d'hydroformylation.

Selon un mode de réalisation particulier de l'invention, on utilise du dioxygène de haute pureté, c'est-à-dire de pureté supérieure à 80 % mol, de préférence supérieure à 90 % mol, et encore plus préférablement supérieure à 99 % mol. De manière alternative, on utilise tout simplement de l'air ou encore de l'air enrichi en dioxygène.

De préférence, l'étape d'oxydation est mise en oeuvre sans ajout de solvant et/ou sans ajout de catalyseur d'activation du dioxygène. On observe une légère amélioration du rendement (on gagne quelques points de rendement) sur les aldéhydes par ajout de traces d'alcalins et/ou d'autres métaux comme catalyseur. Pour cette étape d'oxydation, différents procédés sont utilisables, et notamment ceux décrits dans les documents de brevet US6680395, US6696582, US6800783, US7138544, US7799945, WO10108586, FR2769624. Selon un mode de réalisation particulièrement avantageux du procédé de l'invention, l'étape d'oxydation est réalisée comme dans le document US6696582, sans catalyseur, en deux étapes utilisant deux paliers de températures croissantes successives d'oxydation, notamment pour maîtriser l'exothermie de la réaction. De préférence, l'oxydation est réalisée dans un microréacteur, ce qui présente l'avantage d'évacuer rapidement la chaleur de réaction.

Dans le cas où l'étape d'oxydation est réalisée en présence de catalyseur, on peut notamment utiliser ceux décrits dans les documents de brevet US7799945 et US7138544. Typiquement, on utilise un catalyseur alcalin (généralement sous forme de sel d'acide) et un co-catalyseur pris dans le groupe IV à XII de la classification périodique. Les alcalins améliorent les surfaces de contact gaz-liquide, et par conséquent la migration de l'oxygène dans le milieu. C'est la quantité d'oxygène dissout qui détermine la cinétique de la réaction d'oxydation. L'utilisation de ces catalyseurs lors de l'oxydation n'est toutefois pas essentielle. L'utilisation d'une agitation forte ou d'un microréacteur suffisent généralement pour obtenir un très bon contact gaz liquide, et donc un très bon échange de matière.

De préférence, l'étape d'oxydation est mise en oeuvre sous une pression partielle de dioxygène allant de 1 bar à 50 bar, notamment de 1 bar à 20 bar, de préférence de 1 à 5 bar. Lorsque le dioxygène est sous forme enrichie (à plus forte pression partielle que dans l'air) notamment de pureté supérieure à 80%, la pression partielle de dioxygène injecté dans le milieu réactionnel est de préférence comprise dans la gamme de 5 à 20 bar.

Avantageusement, le dioxygène est injecté en continu dans le milieu réactionnel par bullage, de préférence sous forme d'un flux d'air ou de dioxygène. En effet, le dioxygène injecté sous forme de microbulles favorise le contact gaz-liquide et améliore la vitesse de dissolution dans le liquide. Par «microbulles» on entend des bulles dont le diamètre moyen va de 1 µm à 3 mm, de préférence 100 µm à 3 mm, de préférence de 500 µm à 1 mm. Pour ce faire, toute technique de dispersion peut être utilisée : très forte agitation, turbine autoaspirante, réacteur Loop® de type Buss ChemTech, microréacteur, contacteur à film tombant. Dans ce dernier cas, il peut ne pas y avoir de microbulles, car le transfert de matière s'effectue sans problème. On peut encore utiliser un réacteur dit « Spinning Disk » ou un réacteur dit « Rotating packed bed » idéal pour les milieux visqueux.

Avantageusement, le rapport molaire du dioxygène relativement au produit issu de l'étape d'hydroformylation est compris dans la gamme de 3:2 à 100:2.

Il est plus pratique de parler en rapport molaire par rapport à la stoechiométrie de la réaction d'oxydation, car la réaction est rapide et exothermique. On arrête généralement la réaction lorsqu'on a fait passer du dioxygène en excès, représentant plus de 100% de la stoechiométrie, de préférence plus de 110% de la stoechiométrie, de préférence plus de 120% de la stoechiométrie, voir plus de 220% de la stoechiométrie. Le rapport molaire du dioxygène relativement au nitrile-acide gras est ainsi compris entre (bornes exclues) 100% et 5000% de la stoechiométrie et de préférence supérieur à 110%.

Avantageusement, l'oxydation est mise en oeuvre à une température comprise dans la gamme de 0 °C à 100 °C, de préférence de 20 °C à 100 °C, notamment de 30 °C à 90 °C, de préférence de 40 °C à 80 °C, éventuellement en 2 paliers consécutifs de températures croissantes.

### 3) REDUCTION OU HYDROGENATION DE LA FONCTION NITRILE EN AMINE

L'étape de synthèse des ω-aminoesters ou ω-aminoacides gras à partir, respectivement, des nitrile-esters ou nitrile-acides gras consiste en une réduction ou hydrogénation classique. L'hydrogénation de la fonction nitrile entraîne automatiquement la saturation de la double liaison présente dans la molécule. La réduction de la fonction nitrile en amine primaire est bien connue de l'homme du métier. L'hydrogénation est par exemple conduite en présence de métaux précieux (Pt, Pd, Rh, Ru...) à une température comprise entre 20 et 100 °C sous une pression de 1 à 100 bar, et de préférence de 1 à 50 bar. Elle peut également être conduite en présence de catalyseurs à base de fer, nickel ou cobalt, qui peuvent entraîner des conditions plus sévères avec des températures de l'ordre de 150 °C et des pressions élevées de plusieurs dizaines de bar. Afin de favoriser la formation de l'amine primaire, on opère de préférence avec une pression partielle d'ammoniac. Avantageusement, l'étape de réduction des nitrile-acides gras en ω-amino-acides gras consiste en une hydrogénation utilisant tout catalyseur classique et de préférence les nickel et cobalt de Raney, en particulier le nickel de Raney déposé ou non sur un support tel que la silice.

### SYNTHESE DE POLYAMIDES

Selon un mode de réalisation avantageux, le procédé selon l'invention comprend en outre une étape de synthèse de polyamide par polymérisation utilisant l'ω-aminoacide obtenu à l'étape 3).

En partant d'huile de ricin par exemple, on procède à une méthanolyse pour obtenir le ricinoléate de méthyle de formule :

CH₃-(CH₂)₅-CHOH-CH=CH-(CH₂)₇-COOCH₃

puis on effectue un craquage thermique pour obtenir l'undecylénate de méthyle

CH₂=CH-(CH₂)₈-COOCH₃

qui peut être hydrolysé en acide undécylénique

CH₂=CH-(CH₂)₈-COOH

puis une étape de nitrilation permet d'obtenir l'undécènenitrile

CH₂=CH-(CH₂)₈-CN

De manière alternative, on effectue directement la conversion de l'ester méthylique de l'acide undecylénique en nitrile.

L'undécènenitrile ainsi obtenu est mis en oeuvre dans le procédé de l'invention selon les étapes suivantes :
1) hydroformylation du nitrile en présence de CO et de H₂, pour obtenir un aldéhyde-nitrile à 12 carbones :

   HOC-(CH₂)₁₀-CN
2) autoxydation de l'aldéhyde en acide, pour obtenir :

   HOOC-(CH₂)₁₀-CN
3) réduction du nitrile, pour obtenir l'aminoacide en C12,

   HOOC-(CH₂)₁₀-CH₂-NH₂

   qui permet par polymérisation de fabriquer le polyamide 12 d'origine renouvelable.

En partant d'une huile riche en acide oléique (acide cis-9-octadécénoïque) de formule

CH₃-(CH₂)₇-CH=CH-(CH₂)₇-COOR,

on peut procéder comme suit :
- éthénolyse (métathèse croisée avec l'éthylène ou autre alpha oléfine), pour obtenir :

   CH₃-(CH₂)₇-CH=CH₂ + CH₂=CH-(CH₂)₇-COOR
- méthanolyse et séparation des acides gras pour isoler le décénoate de méthyle

   CH₂=CH-(CH₂)₇-COOCH₃
- hydrolyse de l'ester en acide CH₂=CH-(CH₂)₇-COOH,
- nitrilation de l'acide en décènenitrile CH₂=CH-(CH₂)₇-CN

Puis on soumet le décènenitrile aux étapes suivantes selon le procédé de l'invention :
- hydroformylation du décènenitrile en aldéhyde-nitrile en C11 : HOC-(CH₂)₉-CN
- autoxydation pour former l'acide-nitrile en C11 : HOOC-(CH₂)₉-CN
- réduction pour former l'acide 11-aminoundécanoïque HOOC-(CH₂)₉-CH₂-NH₂.

Par polymérisation de l'acide 11-aminoundécanoïque, on fabrique le polyamide 11. Alternativement, on peut convertir l'acide oléique en nitrile oléique, puis procéder à une éthénolyse (ou autre métathèse croisée avec une alpha-oléfine) pour obtenir le nitrile à 10 atomes de carbone.

En mettant en oeuvre le procédé de l'invention, il est possible de synthétiser toute une gamme d'ω-aminoacides, et donc toute une gamme de polyamides et de polymères.

### Exemples

Sauf indication contraire, tous les pourcentages sont des pourcentages en nombre de moles.

### 1. Matériels

Rh(acac)(CO)₂ (commercialisé par STREM) est utilisé comme précurseur de catalyseur d'hydroformylation. Les phosphines (commercialisées par STREM) sont utilisées telles que réceptionnées ou synthétisées.

### 2. Substrat

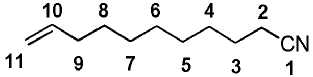

### 3. Réaction d'hydroformylation

Procédure générale : les réactions d'hydroformylation sont effectuées dans des autoclaves en inox de 30 mL. Dans les conditions typiques, une solution dans le toluène (10 mL) de Rh(acac)(CO)₂ (0.001 mmol), phosphine ou diphosphine (0.02 mmol) et d'undécènenitrile (5.0 mmol) est mélangée dans un tube de Schlenk sous atmosphère inerte d'argon pour former une solution homogène. Après agitation à température ambiante pendant 1 h, cette solution est canulée à l'intérieur de l'autoclave préalablement conditionné sous atmosphère inerte. Le réacteur est scellé, purgé plusieurs fois avec un mélange CO/H₂ (1:1), puis pressurisé à 20 bar de ce mélange CO/H₂ à température ambiante, puis chauffé à la température désirée à l'aide d'un bain marie ou d'un bain d'huile. Le milieu réactionnel est agité à l'aide d'un barreau magnétique. Durant la réaction, plusieurs échantillons sont prélevés pour suivre la conversion. Après un temps de réaction approprié, l'autoclave est ramené à température ambiante puis à pression atmosphérique. Le mélange est collecté puis analysé par RMN.

Produits d'hydroformylation dérivés de l'undécènenitrile :

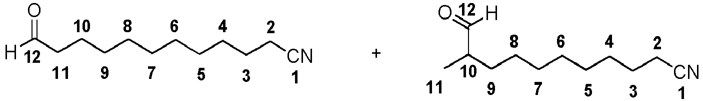

### Exemple 1 : étape d'hydroformylation selon le procédé de l'invention

Une solution de Rh(acac)(CO)₂ (0.25 mg, 0.001 mmol), de Xantphos (11.6 mg, 0.02 mmol) et d'undécènenitrile (826 mg, 5.0 mmol) dans le toluène (10 mL) est préparée dans un tube de Schlenk sous atmosphère inerte d'argon pour former une solution homogène qui est agitée à température ambiante pendant 1 h. Le ratio molaire Xantphos/rhodium est de 20:1 et le ratio molaire substrat/rhodium est de 5000:1. La solution est canulée à l'intérieur d'un autoclave de 30 mL préalablement conditionné sous atmosphère inerte. Le réacteur est scellé, purgé plusieurs fois avec un mélange de gaz CO/H₂ (1:1), puis pressurisé avec 20 bar de CO/H₂ (1:1) à température ambiante puis chauffé à 120 °C. Après 2 h, 48% de l'undécènenitrile a été consommé. Après un temps de réaction de 12 h, le milieu est ramené à température ambiante et à pression atmosphérique. Le mélange est collecté et analysé par RMN. L'analyse montre que la conversion de l'undécénitrile est alors de 99%, qu'il reste une proportion d'oléfine interne de 8%, alors que 92% de produits formés correspondent aux aldéhydes branchés (3%) et linéaire (97%).

### Exemple 2 : étape d'hydroformylation selon le procédé de l'invention

Une solution de Rh(acac)(CO)₂ (0.13 mg, 0.0005 mmol), de Xantphos (5.8 mg, 0.01 mmol) et d'undécènenitrile (826 mg, 5.0 mmol) dans le toluène (10 mL) est préparée dans un tube de Schlenk sous atmosphère inerte d'argon pour former une solution homogène qui est agitée à température ambiante pendant 1 h. Le ratio molaire Xantphos/rhodium est de 20:1 et le ratio molaire substrat/rhodium est de 10 000:1. La solution est canulée à l'intérieur d'un autoclave de 30 mL préalablement conditionné sous atmosphère inerte. Le réacteur est scellé, purgé plusieurs fois avec un mélange de gaz CO/H₂ (1:1), puis pressurisé avec 20 bar de CO/H₂ (1:1) à température ambiante puis chauffé à 120 °C. Après 2 h, 25% de l'undécènenitrile a été consommé. Après un temps de réaction de 12 h, le milieu est ramené à température ambiante et à pression atmosphérique. Le mélange est collecté et analysé par RMN. L'analyse montre que la conversion de l'undécénitrile est de 99%, qu'il reste une proportion d'oléfine interne de 9%, alors que 91% de produits formés correspondent aux aldéhydes branchés (3%) et linéaire (97%).

### Exemple 3 : étape d'hydroformylation selon le procédé de l'invention

Une solution de Rh(acac)(CO)₂ (0.06 mg, 0.0002 mmol), de Xantphos (2.3 mg, 0.004 mmol) et d'undécènenitrile (826 mg, 5.0 mmol) dans le toluène (10 mL) est préparée dans un tube de Schlenk sous atmosphère inerte d'argon pour former une solution homogène qui est agitée à température ambiante pendant 1 h. Le ratio molaire Xantphos/rhodium est de 20:1 et le ratio molaire de substrat/rhodium est de 20 000:1. La solution est canulée à l'intérieur d'un autoclave de 30 mL préalablement conditionné sous atmosphère inerte. Le réacteur est scellé, purgé plusieurs fois avec un mélange de gaz CO/H₂ (1:1), puis pressurisé avec 20 bar de CO/H₂ (1:1) à température ambiante puis chauffé à 140 °C. Après 2 h, 22% de l'undécènenitrile a été consommé. Après un temps de réaction de 18 h, le milieu est ramené à température ambiante et à pression atmosphérique. Le mélange est collecté et analysé par RMN. L'analyse montre que la conversion de l'undécénitrile est alors de 96%, qu'il reste une proportion d'oléfine interne de 6%, alors que 94% des produits formés correspondent aux aldéhydes branchés (4%) et linéaire (96%).

### 4. Auto-oxydation des produits d'hydroformylation dérivés de l'undécènenitrile

Les aldéhydes issus de l'hydroformylation de l'undécènenitrile, obtenus aux exemples 1 à 3 ci-dessus, s'oxydent rapidement en solution dans le toluène et au contact de l'air (exposition à l'air en solution dans le toluène pendant 24 h à température ambiante). Ce constat est confirmé par la disparition du signal carbonyle de la fonction aldéhyde en RMN ¹³C à δ 203 ppm et l'apparition d'un nouveau signal à δ 180 ppm attribuable au carbonyle de la fonction acide carboxylique.

### Réduction des produits d'auto-oxydation, pour obtenir l'aminoacide en C12 :

Un catalyseur Ru/SiC est introduit dans un autoclave en inox équipé d'un agitateur électromagnétique et d'une capacité de 500 mL. Une solution contenant 10 g d'acide 11-cyanoundécanoique et de solvant mixte de 140 mL de n-propanol et de 140 mL d'ammoniaque à 28% en poids d'ammoniac est introduite dans l'autoclave. Après avoir purgé le réacteur plusieurs fois avec de l'azote, le réacteur est pressurisé à 35 bar avec de l'hydrogène. Le réacteur est ensuite chauffé à 110 °C et l'agitation et la température sont maintenues constantes pendant 1,5 h. La réaction ne consomme alors plus d'hydrogène et l'autoclave est redescendu en température jusque 70 °C, puis la pression est réduite à la pression atmosphérique et le liquide incolore est soutiré. Le solvant est ensuite évaporé à environ 60 °C sous vide, et des cristaux blancs (9.7 g) d'acide 12-aminododécanoique sont récupérés.

### 5. Résultats d'hydroformylation de l'undécènenitrile

### 5.1. Avec différents catalyseurs

Plusieurs combinaisons du précurseur Rh(acac)(CO)₂ avec des diphosphines chélatantes, des phosphines ou phosphites sont étudiées pour l'hydroformylation de l'undécènenitrile en milieu organique. Les résultats sont rassemblés dans le Tableau 1. La conversion du substrat en un mélange d'aldéhyde branché (b) et linéaire (l) est observée avec tous les systèmes catalytiques.

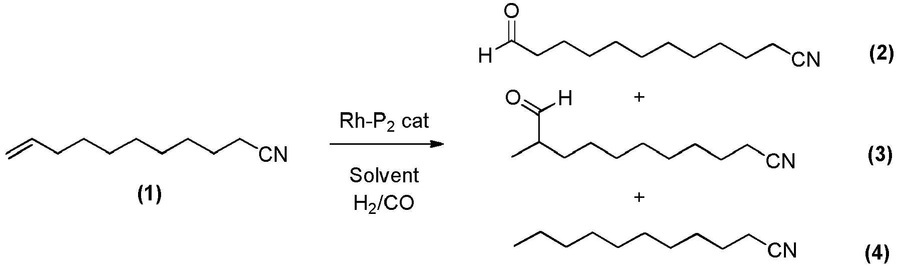

Ces conversions ainsi que la régiosélectivité b/l sont déterminées par spectroscopie RMN.

Avantageusement, l'étape d'hydroformylation du procédé selon l'invention conduit à une sélectivité d'au moins 95%, de préférence d'au moins 97% en aldéhydes linéaires par rapport aux 3 produits (2), (3) et (4).

**Tableau 1 : Hydroformylation de l'undécènenitrile avec différents ligands^{a}**

| **Entrée** | **Ligand [L]** | **Bite angle [L]-[Rh] (°)** | **Conv. (%)^{b}** | **% alcène interne^{c}** | **Sel. (%)^{b}** | | |
|---|---|---|---|---|---|---|---|
| | | | | | **n-2** | **iso-3** | **4** |
| **1** | Dppm | 72 | 5 | 5 | 69 | 31 | nd |
| **2** | Dppe | 85 | 10 | 4 | 46 | 54 | nd |
| **3** | Dppb | 98 | 15 | 4 | 76 | 24 | nd |
| **4** | Xantphos | 107 | 12 | 4 | 97 | 3 | nd |
| **5** | PPh₃ | 0 | 85 | 5 | 71 | 29 | nd |
| **6** | P(OPh)₃, 20 eq | 0 | 85 | 9 | 68 | 32 | nd |
| **7** | P(OPh)₃, 10 eq | 0 | 70 | 8 | 66 | 34 | nd |

| | | | | | | | |
|---|---|---|---|---|---|---|---|
| ^{a} Conditions : [Oléfine] = 0.5M, S/Rh = 5000, L/Rh = 20, 100°C, 20 bar (CO/H2) [1:1], 2h, toluène (10 ml). ^{b} Conversion du nitrile et sélectivité des aldéhydes déterminées par RMN ¹H. nd = non détecté ^{c} % d'alcène interne résiduel ou formé lors de la réaction. | | | | | | | |

Comme indiqué dans le tableau 1 ci-dessus, les systèmes Rh-PPh₃ et Rh-P(OPh)₃ présentent une bonne activité permettant d'atteindre des conversions de 85% en 2 h avec une charge catalytique de 0.2 mol% (entrées 5-7). La meilleure régiosélectivité en faveur de l'aldéhyde linéaire est observée avec le système Rh-Xantphos, lequel permet d'atteindre 97% en faveur de l'aldéhyde linéaire mais avec une activité (conversion) modérée à 100 °C.

### 5.2. Influence des conditions expérimentales

On étudie l'influence des conditions expérimentales sur l'activité et la sélectivité de la réaction d'hydroformylation. Les résultats sont résumés dans le Tableau 2 ci-dessous.

**Tableau 2 : Hydroformylation de l'undécènenitrile avec le ligand Xantphos**

| **Entrée** | **[S]/[Rh]** | **Xantphos (eq)** | **T(°C)** | **Temps (h)** | **Conv. (%)^{b}** | **% alcène interne^{c}** | **Sel. (%)^{b}** | | |
|---|---|---|---|---|---|---|---|---|---|
| | | | | | | | **n-2** | **iso-3** | **4** |
| **1** | 5000 | 20 | 100 | 2 | 12 | 4 | 97 | 3 | nd |
| | | | | 24 | 75 | 6 | 97 | 3 | |
| **2** | 5000 | 20 | 110 | 2 | 26 | 5 | 97 | 3 | nd |
| | | | | 24 | 99 | 8 | 97 | 3 | |
| **3** | 5000 | 20 | 120 | 2 | 48 | 6 | 97 | 3 | nd |
| | | | | 12 | 99 | 8 | 97 | 3 | |
| **4** | 5000 | 20 | 140 | 2 | 59 | 7 | 97 | 3 | nd |
| | | | | 4 | 100 | 10 | 97 | 3 | |
| **5** | 5000 | 10 | 120 | 2 | 48 | 6 | 97 | 3 | nd |
| | | | | 12 | 99 | 7 | 97 | 3 | |
| **6** | 10000 | 20 | 120 | 2 | 25 | 7 | 97 | 3 | nd |
| | | | | 12 | 98 | 9 | 97 | 3 | |
| **7** | 10000 | 10 | 120 | 2 | 21 | 6 | 97 | 3 | nd |
| | | | | 16 | 99 | 9 | 97 | 3 | |
| **8** | 20000 | 20 | 120 | 2 | 5 | 5 | 96 | 4 | nd |
| | | | | 18 | 98 | 8 | 96 | 4 | |
| **9** | 20000 | 20 | 140 | 2 | 22 | 5 | 96 | 4 | nd |
| | | | | 18 | 96 | 6 | 96 | 4 | |
| **10** | 40000 | 20 | 120 | 2 | - | - | 96 | 4 | nd |
| | | | | 18 | 85 | 8 | 96 | 4 | |
| **11** | 80000 | 20 | 110 | 2 | - | - | - | - | nd |
| | | | | 48 | 99 | 13 | 96 | 4 | |
| **12** | 100000 | 20 | 110 | 2 | - | - | - | - | |
| | | | | 96 | 99 | 15 | 96 | 4 | nd |

| | | | | | | | | | |
|---|---|---|---|---|---|---|---|---|---|
| ^{a} Conditions : [Oléfine] = 0.5M, L/Rh = 20, 20 bar, CO/H2 [1:1], toluène (10 ml). ^{b} Conversion du nitrile et sélectivité des aldéhydes déterminées par RMN ¹H; nd = non détecté ^{c} % d'alcène interne résiduel ou formé lors de la réaction | | | | | | | | | |

On constate que la conversion de la réaction augmente logiquement avec la température, sans affecter le ratio l/b (entrées 1-4). Il n'y a pas d'effet marqué en diminuant le ratio ligand/rhodium (entrées 3 et 5). Le ratio substrat/rhodium affecte lui clairement la réaction en termes de conversion et de pourcentage d'alcène interne formé (entrées 3, 6, 8 et 10) ; typiquement, plus la réaction est lente (c'est-à-dire à charge de substrat élevée), plus l'alcène résiduel contient de l'alcène interne provenant de la migration de la double liaison.

Pour un ratio substrat/rhodium de 100 000, une très grande productivité est atteinte à 110 °C avec une chimiosélectivité de 85% et une régiosélectivité de 96% en faveur de l'aldéhyde linéaire (entrée 12).

D'autres systèmes Rh-biphephos permettent d'atteindre des hautes sélectivités (> 97%) ainsi que de bons rendements en aldéhyde linéaire. Les exemples ci-dessous décrivent des tests d'hydroformylation de l'undécènenitrile catalysé par le système Rh-biphephos.

### Procédure générale :

Les réactions d'hydroformylation ont été effectuées dans des autoclaves en inox de 30 mL. Dans les conditions typiques, une solution dans le toluène (10 mL) de Rh(acac)(CO)2 (de 0.001 à 0.0001 mmol), phosphine (de 0.002 à 0.02 mmol) et d'undécènenitrile (5.0 mmol) est mélangée dans un tube de Schlenk sous atmosphère inerte d'argon pour former une solution homogène. Après agitation à température ambiante pendant 1 heure, cette solution est canulée à l'intérieur de l'autoclave préalablement conditionné sous atmosphère inerte. Le réacteur est scellé, purgé plusieurs fois avec un mélange CO / H2 (1:1), puis pressurisé à 20 bar de ce mélange CO / H2 à température ambiante, puis chauffé à la température désirée à l'aide d'un bain marie ou d'un bain d'huile. Durant la réaction, plusieurs échantillons sont prélevés pour suivre la conversion. Après un temps de réaction approprié, l'autoclave est ramené à température ambiante puis à pression atmosphérique. Le mélange est collecté puis analysé par RMN.

**Tableau 3 : Effet de la température pendant l'hydroformylation de l'undécènenitrile^{[a]}**

| Entrée | Temps (°C) | Conv.^{[b]} | Alcènes Internes (%) ^{[c]} | Sel. (%)^{[b]} | |
|---|---|---|---|---|---|
| | | (%) | | **2** | **3** |
| 1 | 80 | 52 | 20 | 99 | 1 |
| 2 | 100 | 75 | 20 | 99 | 1 |
| 3 | 120 | 88 | 22 | 99 | 1 |
| 4 | 140 | 94 | 26 | 99 | 1 |

| | | | | | |
|---|---|---|---|---|---|
| ^{[a]} [undécènenitrile] = 5,0 mmol, [undécènenitrile]/[Rh] = 20000, [biphephos]/[Rh] = 20, toluene = 10 mL, P = 20 bar CO/H₂ (1:1), 4 h. ^{[b]} Conversion du nitrile / sélectivité / % d'alcène interne et **2** et **3** déterminés by RMN ¹H et analyses GLC. ^{[c]} % d'alcène interne, résiduel ou formé lors de la réaction. | | | | | |

Les exemples précédents montrent que le système Rh-biphephos est très actif et sélectif en hydroformylation de l'undécènenitrile (Tableau 3). Dans une gamme de température de 80-120 °C, de bonnes conversions (52-94% de 20,000 équiv) ont été obtenues en 4 h ainsi que d'excellentes sélectivités allant jusqu'à 99%.

Pour pallier le problème éventuel d'isomérisation entraînant la formation d'alcène internes, d'autres expériences sont effectuées en augmentant la durée de réaction, car après conversion totale du substrat, le catalyseur peut isomériser les alcènes internes en alcène terminal et hydroformyler ce dernier en aldéhyde linéaire (Tableau 4).

### Exemple du tableau 4, entrée 4: Hydroformylation du 1,10-undécénitrile (Rh-biphephos avec S/Rh = 20,000 et L/Rh = 20 et recyclage du catalyseur :

Une solution de Rh(acac)(CO)2 dans le toluène (0.65 mg, 0.0025 mmol), de Biphephos (4 mg, 0.005 mmol) et l'undécénitrile (826 mg, 5.0 mmol) est préparée dans un tube de Schlenk sous atmosphère inerte d'argon pour former une solution homogène qui est agitée à température ambiante pendant 1 h. Le ratio molaire Biphephos/rhodium est de 20:1 et le ratio molaire de substrat/rhodium est de 20,000:1. La solution est canulée à l'intérieur d'un autoclave de 30 ml préalablement conditionné sous atmosphère inerte. Le réacteur est scellé, purgé plusieurs fois avec un mélange de gaz CO/H2 (1:1), puis pressurisé avec 20 bar de CO/H2 (1:1) à température ambiante puis chauffé à 120 °C. Après 4 h, 67% de l'undécénitrile a été consommé. Après 24 h, le milieu est ramené à température ambiante et à pression atmosphérique. Le mélange est collecté et analysé par RMN. L'analyse montre que la réaction est totale et qu'il reste une proportion d'oléfine interne de 13%, alors que 87% de produits formés correspondent aux aldéhydes branchés (1%) et linéaire (99%). Si on laisse la réaction se poursuivre, les alcènes internes vont être isomérisés et hydroformylés.

Ainsi après 48 h de réaction, il ne reste plus qu'une proportion d'oléfines internes de 5%, alors que 95% de produits formés correspondent aux aldéhydes branchés (1%) et linéaire (99%).

Une fois la réaction terminée, un recyclage par distillation est effectué, sous atmosphère inerte, en utilisant un système de distillation de type Kugelrohr (« four à boules ») à une température de 180 °C et une pression de 1 mBar. Les produits d'hydroformylation obtenus dans une première fraction sont stables et aucune trace de catalyseur ou ligand résiduel n'a été détectée après analyse RMN (1H et 31P). Le catalyseur contenu en pied de colonne a été réutilisé pour un second run. Pour cela, le catalyseur est réintroduit en Schlenk, avec 10 mL de toluène, une nouvelle charge de Biphephos (0,0005 mmol, 5 equiv) et 5 mmol de 1,10-undécénitrile. La solution est de nouveau canulée à l'intérieur d'un autoclave de 30 mL préalablement conditionné sous atmosphère inerte. Le réacteur est scellé, purgé plusieurs fois avec un mélange de gaz CO/H2 (1:1), puis pressurisé avec 20 bar de CO/H2 (1:1) à température ambiante puis chauffé à 120 °C. Après 48 h, le milieu est alors ramené à température ambiante et à pression atmosphérique. Le mélange est collecté et analysé par RMN. L'analyse montre que la réaction est totale et qu'il reste une proportion d'oléfine interne de 2%, alors que 98% de produits formés correspondent aux aldéhydes branchés (2%) et linéaire (98%). Ce recyclage peut être effectué sur plusieurs cycles sans perte de sélectivité.

### Exemple du tableau 4, entrée 7 : Hydroformylation du 1,10-undécénitrile (Rh-biphephos avec S/Rh = 50,000 et L/Rh = 20 :

Une solution de Rh(acac)(CO)2 dans le toluène (0.65 mg, 0.0025 mmol), de Biphephos (2.0 mg, 0.0025 mmol) et l'undécénitrile (826 mg, 5.0 mmol) est préparée dans un tube de Schlenk sous atmosphère inerte d'argon pour former une solution homogène qui est agitée à température ambiante pendant 1 h. Le ratio molaire Biphephos /rhodium est de 10:1 et le ratio molaire de substrat/rhodium est de 20,000:1. La solution est canulée à l'intérieur d'un autoclave de 30 mL préalablement conditionné sous atmosphère inerte. Le réacteur est scellé, purgé plusieurs fois avec un mélange de gaz CO/H2 (1:1), puis pressurisé avec 20 bar de CO/H2 (1:1) à température ambiante puis chauffé à 120 °C. Après 48 h, le milieu est ramené à température ambiante et à pression atmosphérique. Le mélange est collecté et analysé par RMN. L'analyse montre que la réaction est totale et qu'il reste une proportion d'oléfine interne de 18%, alors que 82% de produits formés correspondent aux aldéhydes branchés (2%) et linéaire (98%). Si on laisse la réaction se poursuivre, les alcènes internes vont être isomérisés et hydroformylés.

**Tableau 4**

| Entrée | [S]/[Rh] | Biphephos (equiv) | Temps (h) | Conv. (%)^{[b]} | Alcènes internes (%)^{[c]} | Sel. (%) ^{[b]} | | | |
|---|---|---|---|---|---|---|---|---|---|
| | | | | | | *n-***2** | *iso-***3** | **4** | **Aldehydes internes** |
| 1 | 10,000 | 20 | 4 | 65 | 28 | 99 | 1 | nd | - |
| | | | 24 | 100 | 12 | 99 | 1 | nd | - |
| 2 | 10,000 | 5 | 4 | 63 | 42 | 99 | 1 | nd | - |
| | | | 24 | 100 | 14 | 99 | 1 | nd | - |
| 3 | 10,000 | 2 | 4 | 58 | 38 | 99 | 1 | nd | - |
| | | | 24 | 100 | 9 | 90 | 4 | nd | 6 |
| 4 | 20,000 | 20 | 4 | 67 | 20 | 99 | 1 | nd | - |
| | | | 24 | 99 | 13 | 99 | 1 | nd | - |
| | | | 48 | 100 | 7 | 99 | 1 | nd | - |
| | | | 72 | 100 | 4 | 99 | 1 | nd | - |
| 5 | 20,000 | 10 | 4 | 81 | 21 | 99 | 1 | nd | - |
| | | | 24 | 99 | 12 | 99 | 1 | nd | - |
| | | | 48 | 100 | 6 | 99 | 1 | nd | - |
| | | | 72 | 100 | 4 | 99 | 1 | nd | - |
| 6 | 20,000 | 5 | 4 | 73 | 23 | 99 | 1 | nd | - |
| | | | 24 | 99 | 17 | 99 | 1 | nd | - |
| | | | 48 | 100 | 11 | 99 | 1 | nd | - |
| | | | 72 | 100 | 6 | 99 | 1 | nd | - |
| 7 | 50,000 | 20 | 4 | 48 | 13 | 99 | 1 | nd | - |
| | | | 72 | 100 | 19 | 99 | 1 | nd | - |
| 8 | 50,000 | 5 | 4 | 65 | 25 | 99 | 1 | nd | - |
| | | | 48 | 100 | 18 | 99 | 1 | nd | - |
| 9 | 100,000 | 20 | 4 | 5 | 16 | 99 | 1 | nd | - |
| | | | 72 | 96 | 27 | 99 | 1 | nd | - |
| 10 | 100,000 | 10 | 4 | 3 | 8 | 99 | 1 | nd | - |
| | | | 24 | 99 | 24 | 98 | 2 | nd | - |
| | | | 96 | 100 | 12 | 92 | 4 | nd | 4 |
| 11 | 100,000 | 5 | 4 | 5 | 8 | 99 | 1 | nd | - |
| | | | 24 | 99 | 45 | 92 | 6 | nd | 2 |
| | | | 96 | 100 | 28 | 86 | 8 | nd | 6 |

| | | | | | | | | | |
|---|---|---|---|---|---|---|---|---|---|
| ^{[a]} [undécènenitrile] = 5,0 mmol, [undécènenitrile]/[Rh] = 20000, [biphephos]/[Rh] = 20, toluene = 10 mL, P = 20 bar CO/H₂ (1:1), 4 h. ^{[b]} Conversion du nitrile / sélectivité / % d'alcène interne et **2** et **3** déterminés by RMN ¹H et analyses GLC. ^{[c]} % d'alcène interne, résiduel ou formé lors de la réaction. nd = non détecté | | | | | | | | | |

Le catalyseur Rh-biphephos peut aussi être recyclé en distillant avec un système de type Kugelrohr (« four à boules »), à 180 °C sous un vide dynamique de 1 mBar, le solvant et les produits organiques (aldéhydes, alcènes internes résiduels). Le résidu solide (avec encore un peu de produits organiques) ainsi récupéré peut ensuite être réutilisé sur plusieurs cycles sans perte importante de sélectivité, après avoir ajouté occasionnellement du ligand frais entre deux cycles pour éviter une modification de l'espèce active en catalyse. Cette procédure est d'abord effectuée en utilisant un ratio S/Rh de 20,000 et un ratio L/Rh de 10 (tableau 5). Ainsi, de très bonnes sélectivités ont été obtenues sur 4 cycles et l'isomérisation/hydroformylation des alcènes internes semble tout aussi efficace car il n'y a pas eu d'accumulation de ces alcènes internes.

**Tableau 5. Hydroformylation de l'undécènenitrile pendant 4 cycles^{[a]}**

| **Entrée^{[a]}** | **Cycle** | **biphephos ajouté (eq)** | **T (°C)** | **Temps (h)** | **Conv**. **(%)^{[b]}** | **Alcènes internes (%) ^{[c]}** | **Sel. (%)^{[b]}** | | |
|---|---|---|---|---|---|---|---|---|---|
| | | | | | | | ***n*-2** | ***iso-* 3** | **aldehydes internes** |
| **1** | I | - | 120 | 24 | 95 | 11 | 99 | 1 | 0 |
| | | | 130 | 48 | 100 | 5 | 99 | 1 | 0 |
| **2^{[d]}** | II | 5 | 120 | 24 | 40 | 12 | 99 | 1 | 0 |
| | | | 130 | 48 | 100 | 2 | 98 | 2 | 0 |
| **3^{[d]}** | III | 5 | 120 | 24 | 35 | 20 | 98 | 2 | 0 |
| | | | 130 | 48 | 100 | 6 | 98 | 2 | 0 |
| **4^{[e]}** | IV | - | 120 | 24 | 26 | 11 | 99 | 1 | 0 |
| | | | 130 | 48 | 90 | 9 | 97 | 3 | 0 |

| | | | | | | | | | |
|---|---|---|---|---|---|---|---|---|---|
| ^{[a]} [undécènenitrile] = 10 mmol, [undécènenitrile]/[Rh] = 20000, [biphephos]/[Rh] = 10, toluene = 5 mL, P = 20 bar CO/H₂ (1:1). ^{[b]} Conversion du nitrile / sélectivité / % d'alcène interne et **2** et **3** déterminés by RMN ¹H et analyses GLC. ^{[c]} % d'alcène interne, résiduel ou formé lors de la réaction. nd = non détecté ^{[d]} Le catalyseur est recyclé sous atmosphère inerte, 5 equiv de biphephos a été ajouté, toluène = 5 mL, P = 20 bar CO/H₂ (1:1). ^{[e]} Le catalyseur est recyclé sous atmosphère inerte, et réutilisé sans ajout de ligand, toluène = 5 mL, P = 20 bar CO/H₂ (1:1). | | | | | | | | | |

Ces résultats prouvent qu'il est facile selon le procédé de l'invention, de recycler ce catalyseur par voie de distillation. D'autres tests sont effectués en augmentant le ratio S/Rh à 100,000 avec un ratio L/Rh plus élevé (20 initialement). Le tableau 6 montre que cette procédure fonctionne sans grande diminution de l'activité ; la productivité est très bonne puisqu'elle atteint près de 200,000 sur deux cycles (le premier + le recyclage), ceci en n'utilisant que 25 équivalents de biphephos.

### Exemples du tableau 6, entrées 1 et 2 : Hydroformylation du 1,10-undécénitrile (Rh-biphephos avec S/Rh = 100,000 et L/Rh = 20 ; et recyclage du catalyseur :

Une solution de Rh(acac)(CO)2 dans le toluène (0.026 mg, 0.0001 mmol), de Biphephos (1.6 mg, 0.002 mmol) et l'undécénitrile (1,65 g, 10.0 mmol) est préparée dans un tube de Schlenk sous atmosphère inerte d'argon pour former une solution homogène qui est agitée à température ambiante pendant 1 h. Le ratio molaire Biphephos /rhodium est de 20:1 et le ratio molaire de substrat/rhodium est de 100,000:1. La solution est canulée à l'intérieur d'un autoclave de 30 mL préalablement conditionné sous atmosphère inerte. Le réacteur est scellé, purgé plusieurs fois avec un mélange de gaz CO/H2 (1:1), puis pressurisé avec 20 bar de CO/H2 (1:1) à température ambiante puis chauffé à 120 °C. Après 48 h, 85% de l'undécénitrile a été consommé. On ajuste ensuite la température à 130 °C, sur un temps de réaction de 48 h supplémentaire et le milieu est ramené à température ambiante et à pression atmosphérique. Le mélange est collecté et analysé par RMN. L'analyse montre que la réaction est totale et qu'il reste une proportion d'oléfine interne de 9%, alors que 91% de produits formés correspondent aux aldéhydes branchés (2%) et linéaire (98%).

Une fois la réaction terminée, un recyclage par distillation est effectué, sous atmosphère inerte, en utilisant un système de distillation de type Kugelrohr à une température de 180 °C et un vide dynamique de 1 mBar. Les produits d'hydroformylation obtenus dans une première fraction sont stables et aucune trace de catalyseur ou ligand résiduel n'est détectée après analyse RMN (1H et 31P). Le catalyseur contenu en pied de colonne est réutilisé pour un second run. Pour cela, le catalyseur est réintroduit en Schlenk, avec 10 mL de toluène, une nouvelle charge fraîche de Biphephos (0,5 □mol, 5 equiv) et 10 mmol de 1,10-undécénitrile. La solution est de nouveau canulée à l'intérieur d'un autoclave de 30 mL préalablement conditionné sous atmosphère inerte. Le réacteur est scellé, purgé plusieurs fois avec un mélange de gaz CO/H2 (1:1), puis pressurisé avec 20 bar de CO/H2 (1:1) à température ambiante puis chauffé à 120 °C. Après 48 h, 72% de l'undécénitrile est consommé. On ajuste ensuite la température à 130 °C, sur un temps de réaction de 48 h supplémentaire et le milieu est ramené à température ambiante et à pression atmosphérique. Le mélange est collecté et analysé par RMN. L'analyse montre que la réaction est totale et qu'il reste une proportion d'oléfines internes de 11%, alors que 89% des produits formés correspondent aux aldéhydes branchés (2%) et linéaire (98%).

**Tableau 6. Hydroformylation de l'undécènenitrile sur 2 cycles à hauts rapports S/Rh^{[a]}**

| **Entrée^{[a]}** | **Cycle** | **Biphephos ajouté (eq)** | **T(°C)** | **Time (h)** | **Conv. (%)^{[b]}** | **% alcènes internes^{[c]}** | **Sel. (%)^{[b]}** | | |
|---|---|---|---|---|---|---|---|---|---|
| | | | | | | | *n****-*2** | *iso-***3** | **ss-pdt** |
| **1** | Cycle I | - | 120 | 48 | 85 | 23 | 99 | 1 | - |
| | | | 130 | 96 | 100 | 9 | 98 | 2 | - |
| **2**^{[d]} | Cycle II | 5 | 120 | 48 | 72 | 24 | 99 | 1 | - |
| | | | 130 | 96 | 100 | 11 | 98 | 2 | - |

| | | | | | | | | | |
|---|---|---|---|---|---|---|---|---|---|
| ^{[a]} [undécènenitrile] = 10 mmol, [undécènenitrile]/[Rh] = 100000, [biphephos]/[Rh] = 20, toluene = 10 mL, P = 20 bar CO/H₂ (1:1). ^{[b]} Conversion du nitrile / sélectivité / % d'alcène interne déterminés by RMN ¹H et analyses GLC. ^{[c]} % d'alcène interne, résiduel ou formé lors de la réaction. ^{[d]} Le catalyseur est recyclé sous atmosphère inerte, 5 equiv de biphephos a été ajouté, toluène = 10 mL, P = 20 bar CO/H₂ (1:1). | | | | | | | | | |

### Exemple comparatif avec le Methyl-10-undécénoate

D'autres tests sont effectués afin de comparer les performances du système Rh-biphephos sur le undécènenitrile et le méthyle 10-undécénoate (Tableau 7). Les essais sont effectués avec du undécénoate de méthyle qui a été préalablement distillé. Au vu des résultats (notamment en comparant l'entrée 4 du tableau 7 avec l'entrée 1 du Tableau 6), il apparait que le système Rh-biphephos est moins sélectif et moins actif vis-à-vis du méthyle 10-undécénoate qu'avec le nitrile gras.

### Exemple du tableau 7, entrée 4 : Hydroformylation du méthyle 10-undécénoate (Rh-biphephos avec S/Rh = 100,000 et L/Rh = 20 :

Cet essai est réalisé dans les mêmes conditions que celles décrites ci-dessus dans l'exemple du tableau 6 en remplaçant le 1,10-undécénitrile par du méthyle 10-undécénoate. Il n'y a pas de recyclage du catalyseur dans ce cas. Après 48 h, 68% du méthyle 10-undécénoate a été consommé. Un prélèvement est collecté et analysé par RMN. L'analyse montre qu'il reste une proportion d'oléfine interne de 45%, alors que 23% de produits formés correspondent aux aldéhydes branchés (15%) et linéaire (85%).

**Tableau 7. Hydroformylation du méthyle 10-undécénoate ^{[a]}**

| **Entrée** | **S/Rh** | **L/Rh** | **T (°C)** | **Temps (h)** | **Conv. (%)^{[b]}** | **% alcènes internes^{[c]}** | **Sel. (%)^{[b]}** | | |
|---|---|---|---|---|---|---|---|---|---|
| | | | | | | | *n***-2** | *iso-***3** | **ss-pdt** |
| **1** | 2,000 | 10 | 100 | 24 | 100 | 10 | 96 | 4 | - |
| **2** | 5,000 | 10 | 120 | 24 | 100 | 15 | 95 | 3 | 2 |
| **3** | 20,000 | 10 | 120 | 48 | 93 | 12 | 90 | 5 | 5 |
| **4** | 100,000 | 20 | 120 | 48 | 68 | 45 | 85 | 12 | 3 |

| | | | | | | | | | |
|---|---|---|---|---|---|---|---|---|---|
| ^{[a]} méthyl 10-undécènenitrile distillé = 5,0 mmol, toluène = 10 mL, P = 20 bar CO/H₂ (1:1). ^{[b]} Conversion du méthyl 10-undécènoate / sélectivité / % d'alcène interne déterminés by RMN ¹H et analyses GLC. ^{[c]} % d'alcène interne, résiduel ou formé lors de la réaction. | | | | | | | | | |

En définitive, le procédé selon l'invention présente de nombreux avantages par rapport aux procédés existants. Il est simple à mettre en oeuvre. Il ne nécessite pas d'équipement particulier et peut être mis en oeuvre dans les dispositifs industriels existants, ce qui permet de démarrer une commercialisation, même avec de petits tonnages en capacité de production. Il ne nécessite pas non plus de matières premières ni de catalyseurs coûteux. Le procédé selon l'invention est versatile puisqu'il permet d'utiliser une large gamme de matières premières, il n'est pas lié à une huile en particulier.

Contrairement aux procédés de l'art antérieur qui utilisent nécessairement une étape de métathèse, et conduisent à un aminoester dans les cas où les matières premières sont par exemple un ester gras et de l'acrylonitrile, ou un nitrile gras et de l'acrylate de méthyle, on obtient selon le procédé de l'invention un aminoacide. De plus, le nombre de carbones d'origine non-renouvelable de l'aminoacide obtenu selon le procédé de l'invention est nul, ou alors limité à 1 dans le cas où le gaz de synthèse (CO) utilisé lors de l'hydroformylation n'est pas produit à partir de biomasse.

## Revendications

1. Procédé de synthèse d'un composé ω-aminoacide de formule
HOOC-(CH₂)ᵣ₊₂-CH₂NH₂ où 4 ≤ r ≤ 13,
comprenant :
1) une étape d'hydroformylation d'un nitrile gras insaturé choisi parmi les composés de formule :
CH₂=CH-(CH₂)ᵣ-CN
et leurs mélanges,
en faisant réagir ledit nitrile avec du monoxyde de carbone et du dihydrogène de façon à obtenir au moins un aldéhyde nitrile gras de formule :
HOC-(CH₂)ᵣ₊₂-CN,
2) une étape d'oxydation en présence de dioxygène, au cours de laquelle l'aldéhyde-nitrile obtenu à l'étape 1) est converti en acide-nitrile gras de formule :
HOOC-(CH₂)ᵣ₊₂-CN,
3) une étape de réduction au cours de laquelle l'acide-nitrile obtenu à l'étape 2) est converti en ω-aminoacide de formule :
HOOC-(CH₂)ᵣ₊₂-CH₂NH₂.
dans lequel l'étape d'hydroformylation est catalysée en présence d'un système catalyseur comprenant :
- au moins un métal choisi parmi le rhodium, le palladium, le cobalt, le ruthénium et leurs mélanges; et
- au moins un ligand bidentate diphosphine chélatante ou ligand monodentate de type monophosphine ou monophosphite.

2. Procédé selon la revendication 1 comprenant en outre une étape de métathèse catalytique croisée avec un alcène choisi parmi l'éthylène, réalisée sur le nitrile gras avant l'étape 1) de façon à fabriquer un nitrile gras oméga-insaturé répondant à la formule CH₂=CH-(CH₂)ᵣ-CN.

3. Procédé selon la revendication 1, comprenant en outre une étape de fabrication dudit nitrile gras à partir d'un acide ou ester gras insaturé d'origine naturelle de formule :
(CH₂=CH-(CH₂)ᵣ-COO-)ₚ-G, où
p est un indice entier tel que 1 ≤ p ≤ 3
G est H, un radical alkyle de 1 à 11 atomes de carbone ou un radical comportant 2 ou 3 atomes de carbone porteur de 1 ou 2 fonctions(s) hydroxyle(s),
ladite fabrication comprenant l'ammoniation de la fonction carbonyle de l'acide ou ester gras insaturé d'origine naturelle en fonction nitrile.

4. Procédé selon la revendication 3 comprenant en outre, avant ammoniation :
- soit une métathèse catalytique croisée avec de l'éthylène réalisée sur l'acide ou l'ester gras insaturé d'origine naturelle
- soit une pyrolyse de l'acide ou ester gras insaturé d'origine naturelle suivie de distillation, puis éventuellement hydrolyse en acide dans le cas de l'ester,
de façon à fabriquer un acide ou ester gras oméga-insaturé de formule :
CH₂=CH-(CH₂)ᵣ-COOR₂, R₂ est H ou un radical alkyle en C2-4,
et de sorte qu'à l'issue de l'étape d'ammoniation réalisée sur cet acide ou ester gras oméga-insaturé, on obtient un nitrile gras oméga-insaturé de formule CH₂=CH-(CH₂)ᵣ-CN.

5. Procédé selon la revendication 3 ou 4, dans lequel l'acide (ou ester) gras insaturé d'origine naturelle est un acide (ou ester) hydroxylé, de préférence l'acide (ou l'ester) ricinoléique ou l'acide (ou l'ester) lesquerollique, plus préférablement l'acide ricinoléique.

6. Procédé selon l'une quelconque des revendications précédentes, dans lequel le système comprend au moins une phosphine, un phosphite ou une diphosphine chélatante choisie parmi : 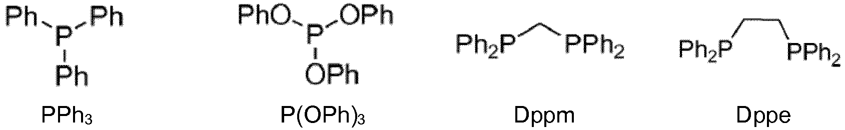 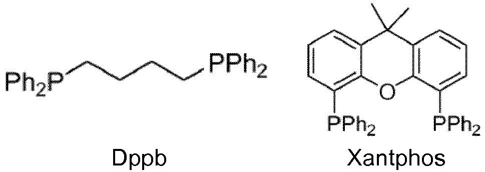 .

7. Procédé selon la revendication 6, dans lequel le métal du système est apporté sous la forme d'un précurseur comprenant ledit métal et au moins un composé choisi parmi des acétylacétonates, des composés carbonyles, et leurs mélanges.

8. Procédé selon l'une quelconque des revendications 6 ou 7, dans lequel le système catalyseur d'hydroformylation comprend du rhodium, et/ou du ruthénium.

9. Procédé selon l'une quelconque des revendications 6 à 8 dans lequel l'hydroformylation est catalysée par un système choisi parmi : Rh-PPh₃, Rh-P(OPh)₃, Rh-Xantphos, et leurs mélanges.

10. Procédé selon l'une quelconque des revendications 6 à 9, dans lequel le ratio molaire [ligand]/[métal] est compris dans la gamme de 60:1 à 1:1.

11. Procédé selon l'une quelconque des revendications précédentes dans lequel l'hydroformylation est réalisée en milieu organique.

12. Procédé selon l'une quelconque des revendications précédentes dans lequel l'hydroformylation est réalisée à une température comprise dans la gamme de 70 à 150

13. Procédé selon l'une quelconque des revendications précédentes dans lequel l'hydroformylation est réalisée pendant une durée allant de 2 à 24 heures.

14. Procédé selon l'une quelconque des revendications précédentes dans lequel l'hydroformylation est réalisée sous pression partielle de CO/H₂ comprise dans la gamme de 5 à 50 bar de CO/H₂, et selon un ratio CO:H₂ compris dans la gamme de 1:3 à 3:1.

15. Procédé selon l'une quelconque des revendications précédentes dans lequel le ratio [Substrat]/[Métal] est compris dans la gamme de 5 000 à 500 000.

16. Procédé selon l'une quelconque des revendications précédentes dans lequel l'étape d'oxydation est mise en oeuvre en faisant barboter du dioxygène ou un mélange gazeux contenant du dioxygène dans le produit résultant de l'hydroformylation, éventuellement en présence du catalyseur d'hydroformylation.

17. Procédé selon l'une des revendications précédentes dans lequel l'étape d'oxydation est mise en oeuvre sans ajout de solvant et/ou sans ajout de catalyseur d'activation du dioxygène.

18. Procédé selon l'une des revendications précédentes dans lequel l'étape d'oxydation est mise en oeuvre sous une pression partielle de dioxygène allant de 1 bar à 50 bar.

19. Procédé selon l'une des revendications précédentes dans lequel le dioxygène est injecté en continu dans le milieu réactionnel par bullage, de préférence sous forme d'un flux d'air ou d'oxygène, injecté de préférence en excès par rapport à la stoechiométrie de la réaction d'oxydation.

20. Procédé selon l'une des revendications précédentes dans lequel le rapport molaire du dioxygène relativement au produit issu de l'étape d'hydroformylation est compris dans la gamme de 3:2 à 100:2.

21. Procédé selon l'une des revendications précédentes dans lequel l'oxydation est mise en oeuvre à une température comprise dans la gamme de 0 °C à 100 °C, éventuellement en 2 paliers consécutifs de températures croissantes.

22. Procédé selon l'une quelconque des revendications précédentes comprenant en outre une étape de synthèse de polyamide par polymérisation utilisant l'ω-aminoacide obtenu à l'étape 3).

23. Procédé selon l'une quelconque des revendications 6 à 22, dans lequel l'étape d'hydroformylation comprend le recyclage du système catalyseur d'hydroformylation, éventuellement complété par un apport de catalyseur et/ou de ligand neuf lors d'un cycle ultérieur d'hydroformylation.

24. Procédé selon la revendication 23, dans lequel le système catalyseur recyclé est obtenu par évaporation au moins partielle de solvant et/ou d'aldéhyde-nitrile et ou de réactif n'ayant pas réagi.

## Patentansprüche

1. Verfahren zur Synthese einer ω-Aminosäure-Verbindung der Formel
HOOC-(CH₂)ᵣ₊₂-CH₂NH₂,
wobei 4 ≤ r ≤ 13, umfassend:
1) einen Schritt der Hydroformylierung eines ungesättigten Fettnitrils, das aus den Verbindungen der Formel:
CH₂=CH-(CH₂)ᵣ-CN
und Mischungen davon ausgewählt ist,
durch Umsetzung des Nitrils mit Kohlenmonoxid und Diwasserstoff zum Erhalt mindestens eines Fettnitrilaldehyds der Formel:
HOC-(CH₂)ᵣ₊₂-CN,
2) einen Schritt der Oxidation in Gegenwart von Disauerstoff, in dessen Verlauf der in Schritt 1) erhaltene Nitrilaldehyd in eine Fettnitrilsäure der Formel:
HOOC-(CH₂)ᵣ₊₂-CN
umgewandelt wird,
3) einen Schritt der Reduktion, in dessen Verlauf die in Schritt 2) erhaltene Nitrilsäure in eine ω-Aminosäure der Formel:
HOOC-(CH₂)ᵣ₊₂-CH₂NH₂
umgewandelt wird,
wobei der Schritt der Hydroformylierung in Gegenwart des Katalysatorsystems, umfassend:
- mindestens ein Metall, das aus Rhodium, Palladium, Cobalt, Ruthenium und Mischungen davon ausgewählt ist; und
- mindestens einen chelatbildenden zweizähnigen Diphosphin-Liganden oder einzähnigen Liganden vom Monophosphin- oder Monophosphit-Typ;
katalysiert wird.

2. Verfahren nach Anspruch 1, außerdem umfassend einen Schritt der katalytischen Kreuzmetathese mit einem aus Ethylen ausgewählten Alken, der vor Schritt 1) an dem Fettnitril durchgeführt wird, um ein omega-ungesättigtes Fettnitril der Formel CH₂=CH-(CH₂)ᵣ-CN herzustellen.

3. Verfahren nach Anspruch 1, außerdem umfassend einen Schritt der Herstellung des Fettnitrils aus einer ungesättigten Fettsäure oder einem ungesättigten Fettsäureester natürlichen Ursprungs der Formel:
(CH₂=CH-(CH₂)ᵣ-COO-)ₚ-G,
wobei
p für einen solchen ganzzahligen Index steht, dass 1 ≤ p ≤ 3,
G für H, einen Alkylrest mit 1 bis 11 Kohlenstoffatomen oder einen Rest mit 2 oder 3 Kohlenstoffatomen, der 1 oder 2 Hydroxylfunktionen trägt, steht,
wobei die Herstellung die Ammonisierung der Carbonylfunktion der ungesättigten Fettsäure bzw. des ungesättigten Fettsäureesters natürlichen Ursprungs zu einer Nitrilfunktion umfasst.

4. Verfahren nach Anspruch 3, außerdem umfassend vor der Ammonisierung:
- entweder eine katalytische Kreuzmetathese mit Ethylen, die an der ungesättigten Fettsäure bzw. dem ungesättigten Fettsäureester natürlichen Ursprungs durchgeführt wird,
- oder eine Pyrolyse der ungesättigten Fettsäure bzw. des ungesättigten Fettsäureesters natürlichen Ursprungs mit anschließender Destillation und dann gegebenenfalls Hydrolyse zu einer Säure im Fall des Esters,
um eine omega-ungesättigte Fettsäure bzw. einen omega-ungesättigten Fettsäureester der Formel: CH₂=CH-(CH₂)ᵣ-COOR₂, wobei R₂ für H oder einen C₂₋₄-Alkylrest steht,
herzustellen, und derart, dass man nach dem an dieser omega-ungesättigten Fettsäure bzw. diesem omega-ungesättigten Fettsäureester durchgeführten Ammonisierungsschritt ein omega-ungesättigtes Fettnitril der Formel CH₂=CH-(CH₂)ᵣ-CN erhält.

5. Verfahren nach Anspruch 3 oder 4, wobei es sich bei der ungesättigten Fettsäure (bzw. dem ungesättigten Fettsäureester) natürlichen Ursprungs um eine Hydroxysäure (bzw. einen Hydroxyester), vorzugsweise Ricinolsäure (bzw. Ricinolsäureester) oder Lesquerolsäure (bzw. Lesquerolsäureester), weiter bevorzugt Ricinolsäure, handelt.

6. Verfahren nach einem der vorhergehenden Ansprüche, wobei das System mindestens ein Phosphin, ein Phosphit oder ein chelatbildendes Diphosphin umfasst, das aus: 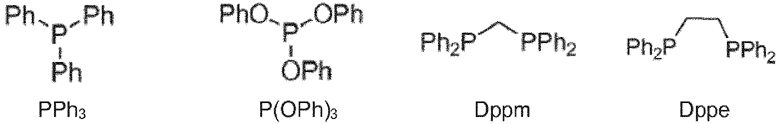 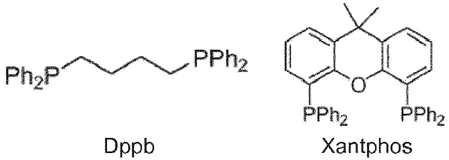 ausgewählt ist.

7. Verfahren nach Anspruch 6, wobei das Metall des Systems in Form einer Vorstufe bereitgestellt wird, die das Metall und mindestens eine Verbindung, die aus Acetylacetonaten, Carbonylverbindungen und Mischungen davon ausgewählt ist, umfasst.

8. Verfahren nach Anspruch 6 oder 7, wobei das Hydroformylierungskatalysatorsystem Rhodium und/oder Ruthenium umfasst.

9. Verfahren nach einem der Ansprüche 6 bis 8, wobei die Hydroformylierung durch ein System katalysiert wird, das aus Rh-PPh₃, Rh-P(OPh)₃ und Rh-Xantphos und Mischungen davon ausgewählt ist.

10. Verfahren nach einem der Ansprüche 6 bis 9, wobei das [Ligand] / [Metall]-Molverhältnis im Bereich von 60:1 bis 1:1 liegt.

11. Verfahren nach einem der vorhergehenden Ansprüche, wobei die Hydroformylierung in einem organischen Medium durchgeführt wird.

12. Verfahren einem der vorhergehenden Ansprüche, wobei die Hydroformylierung bei einer Temperatur im Bereich von 70 bis 150 °C durchgeführt wird.

13. Verfahren nach einem der vorhergehenden Ansprüche, wobei die Hydroformylierung über einen Zeitraum im Bereich von 2 bis 24 Stunden durchgeführt wird.

14. Verfahren nach einem der vorhergehenden Ansprüche, wobei die Hydroformylierung unter einem CO/H₂-Partialdruck im Bereich von 5 bis 50 bar CO/H₂ und gemäß einem CO:H₂-Verhältnis im Bereich von 1:3 bis 3:1 durchgeführt wird.

15. Verfahren nach einem der vorhergehenden Ansprüche, wobei das [Substrat] / [Metall]-Verhältnis im Bereich von 5000 bis 500.000 liegt.

16. Verfahren einem der vorhergehenden Ansprüche, wobei der Oxidationsschritt durch Durchleiten von Disauerstoff oder einer Disauerstoff enthaltenden Gasmischung durch das Produkt aus der Hydroformylierung, gegebenenfalls in Gegenwart des Hydroformylierungskatalysators, durchgeführt wird.

17. Verfahren nach einem der vorhergehenden Ansprüche, wobei der Oxidationsschritt ohne Zugabe von Lösungsmitteln und/oder ohne Zugabe von Disauerstoffaktivierungskatalysator durchgeführt wird.

18. Verfahren nach einem der vorhergehenden Ansprüche, wobei der Oxidationsschritt unter einem Disauerstoff-Partialdruck im Bereich von 1 bar bis 50 bar durchgeführt wird.

19. Verfahren nach einem der vorhergehenden Ansprüche, wobei der Disauerstoff durch Einblasen kontinuierlich in das Reaktionsmedium eingeleitet wird, vorzugsweise in Form eines Luft- oder Sauerstoffstroms, der vorzugsweise im Überschuss bezüglich der Stöchiometrie der Oxidationsreaktion eingeleitet wird.

20. Verfahren einem der vorhergehenden Ansprüche, wobei das Molverhältnis von Disauerstoff zu dem Produkt aus dem Hydroformylierungsschritt im Bereich von 3:2 bis 100:2 liegt.

21. Verfahren einem der vorhergehenden Ansprüche, wobei die Oxidation bei einer Temperatur im Bereich von 0 °C bis 100 °C, gegebenenfalls in 2 aufeinanderfolgenden Stufen mit zunehmenden Temperaturen, durchgeführt wird.

22. Verfahren nach einem der vorhergehenden Ansprüche, außerdem umfassend einen Schritt der Synthese von Polyamid durch Polymerisation unter Verwendung der in Schritt 3) erhaltenen ω-Aminosäure.

23. Verfahren nach einem der Ansprüche 6 bis 22, wobei der Hydroformylierungsschritt das Rezyklieren des Hydroformylierungskatalysatorsystems, gegebenenfalls ergänzt durch einen Eintrag von frischem Katalysator und/oder Liganden bei einem späteren Hydroformylierungszyklus, umfasst.

24. Verfahren nach Anspruch 23, wobei das rezyklierte Katalysatorsystem durch zumindest teilweises Abdampfen von Lösungsmittel und/oder Nitrilaldehyd und/oder nicht umgesetztem Reagenz erhalten wird.

## Claims

1. Process for synthesizing an **ω**-amino acid compound of formula
HOOC-(CH₂)ᵣ₊₂-CH₂NH₂,
where, 4 ≤ r ≤ 13 comprising:
1) a step of hydroformylation of an unsaturated fatty nitrile chosen from the compounds of formula:
CH₂=CH-(CH₂)ᵣ-CN,
and mixtures thereof,
by reacting said nitrile with carbon monoxide and dihydrogen so as to obtain at least one fatty nitrile aldehyde of formula:
HOC-(CH₂)ᵣ₊₂-CN,
2) a step of oxidation in the presence of dioxygen, during which the nitrile-aldehyde obtained in step 1) is converted into a fatty nitrile-acid of formula:
HOOC-(CH₂)ᵣ₊₂-CN,
3) a reduction step during which the nitrile-acid obtained in step 2) is converted into an **ω**-amino acid of formula:
HOOC-(CH₂)ᵣ₊₂-CH₂NH₂,
wherein the hydroformylation step is catalyzed in the presence of a catalyst system comprising:
- at least one metal chosen from rhodium, cobalt and ruthenium, and mixtures thereof; and
- at least one chelating diphosphine bidentate ligand or monodentate ligand of the monophosphine or monophosphite type.

2. Process according to Claim 1, also comprising a step of catalytic cross metathesis with an alkene chosen from ethylene,
carried out on the fatty nitrile before step 1) so as to produce an omega-unsaturated fatty nitrile corresponding to the formula CH₂=CH-(CH₂)ᵣ-CN.

3. Process according to Claim 1, also comprising a step of producing said fatty nitrile from an unsaturated fatty acid or ester of natural origin of formula:
(CH₂=CH-(CH₂)ᵣ-COO-)ₚ-G, where
p is an integer index such that 1 ≤ p ≤ 3,
G is H, an alkyl radical having from 1 to 11 carbon atoms or a radical comprising 2 or 3 carbon atoms bearing 1 or 2 hydroxyl function(s),
said production comprising the ammoniation of the carbonyl function of the unsaturated fatty acid or ester of natural origin to give a nitrile function.

4. Process according to Claim 3, also comprising, before ammoniation:
- either a catalytic cross metathesis with ethylene carried out on the unsaturated fatty acid or ester of natural origin,
- or a pyrolysis of the unsaturated fatty acid or ester of natural origin, followed by distillation, then optionally hydrolysis to give an acid in the case of the ester,
so as to produce an omega-unsaturated fatty acid or ester of formula: CH₂=CH-(CH₂)ᵣ-COOR₂, R₂ being H or a C₂-C₄ alkyl radical, and such that, after the ammoniation step carried out on this omega-unsaturated fatty acid or ester, an omega-unsaturated fatty nitrile of formula CH₂=CH-(CH₂)ᵣ-CN is obtained.

5. Process according to Claim 3 or 4, wherein the unsaturated fatty acid (or ester) of natural origin is a hydroxylated acid (or ester), preferably ricinoleic acid (or ester) or lesquerolic acid (or ester), more preferably ricinoleic acid.

6. Process according to any one of the preceding claims, wherein the system comprises at least one phosphine, one phosphite or one chelating diphosphine chosen from:

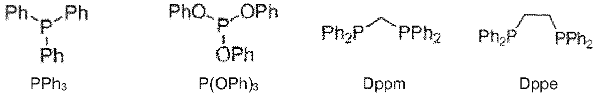

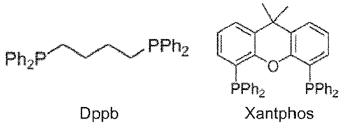

7. Process according to Claim 6, wherein the metal of the system is provided in the form of a precursor comprising said metal and at least one compound chosen from acetylacetonates, carbonyl compounds, and mixtures thereof.

8. Process according to either one of Claims 6 and 7, wherein the hydroformylation catalyst system comprises rhodium and/or ruthenium.

9. Process according to any one of Claims 6 to 8, wherein the hydroformylation is catalyzed by a system chosen from: Rh-PPh₃, Rh-P(OPh)₃ and Rh-Xantphos, and mixtures thereof.

10. Process according to any one of Claims 6 to 9, wherein the [ligand] / [metal] molar ratio is included in the range of from 60:1 to 1:1.

11. Process according to any one of the preceding claims, wherein the hydroformylation is carried out in an organic medium.

12. Process according to any one of the preceding claims, wherein the hydroformylation is carried out at a temperature included in the range of from 70 to 150°C.

13. Process according to any one of the preceding claims, wherein the hydroformylation is carried out for a period of time ranging from 2 to 24 hours.

14. Process according to any one of the preceding claims, wherein the hydroformylation is carried out at a partial CO/H₂ pressure included in the range of from 5 to 50 bar of CO/H₂, and according to a CO:H₂ ratio included in the range of from 1:3 to 3:1.

15. Process according to any one of the preceding claims, wherein the [Substrate] / [Metal] ratio is included in the range of from 5000 to 500 000.

16. Process according to any one of the preceding claims, wherein the oxidation step is carried out by sparging dioxygen or a dioxygen-containing gas mixture in the product resulting from the hydroformylation, optionally in the presence of the hydroformylation catalyst.

17. Process according to one of the preceding claims, wherein the oxidation step is carried out without the addition of solvent and/or without the addition of dioxygen activation catalyst.

18. Process according to one of the preceding claims, wherein the oxidation step is carried out at a partial dioxygen pressure ranging from 1 bar to 50 bar.

19. Process according to one of the preceding claims, wherein the dioxygen is continuously injected into the reaction medium by bubbling, preferably in the form of an air or oxygen stream, preferably injected in excess with respect to the stoichiometry of the oxidation reaction.

20. Process according to one of the preceding claims, wherein the molar ratio of the dioxygen relative to the product resulting from the hydroformylation step is included in the range of from 3:2 to 100:2.

21. Process according to one of the preceding claims, wherein the oxidation is carried out at a temperature included in the range of from 0°C to 100°C, optionally in 2 consecutive increasing stationary temperature phases.

22. Process according to any one of the preceding claims, also comprising a step of synthesis of polyamide by polymerization using the **ω**-amino acid obtained in step 3).

23. Process according to any one of Claims 6 to 22, wherein the hydroformylation step comprises the recycling of the hydroformylation catalyst system, optionally supplemented by a provision of new catalyst and/or ligand during a subsequent hydroformylation cycle.

24. Process according to Claim 23, wherein the recycled catalyst system is obtained by at least partial evaporation of solvent and/or of nitrile-aldehyde and/or of unreacted reagent.
